(19) Europäisches Patentamt
European Patent Office
Office européen des brevets

(11) Veröffentlichungsnummer: 0 023 706
B1

(12) EUROPÄISCHE PATENTSCHRIFT

(45) Veröffentlichungstag der Patentschrift: 02.03.83

(51) Int. Cl.³: C 07 D 211/52, C 07 D 211/48, C 07 D 211/94, A 61 K 31/445

(21) Anmeldenummer: 80104543.6

(22) Anmeldetag: 01.08.80

(54) **4-Phenoxypiperidine, Verfahren zu ihrer Herstellung, ihre Anwendung und sie enthaltende Arzneimittel.**

(30) Priorität: **03.08.79 CH 7127/79**

(43) Veröffentlichungstag der Anmeldung: **11.02.81 Patentblatt 81/6**

(45) Bekanntmachung des Hinweises auf die Patenterteilung: **02.03.83 Patentblatt 83/9**

(84) Benannte Vertragsstaaten: **AT BE CH DE FR GB IT LI LU NL SE**

(56) Entgegenhaltungen:
**DE-A-2 735 051**
**DE-A-2 822 307**
**DE-B-1 289 529**
**FR-M-1 799**
**US-A-3 438 991**
**US-A-3 506 671**
**US-A-3 551 433**

**Die Akte enthält technische Angaben, die nach dem Eingang der Anmeldung eingereicht wurden und die nicht in dieser Patentschrift enthalten sind.**

(73) Patentinhaber: **Byk Gulden Lomberg Chemische Fabrik GmbH, Byk-Gulden-Strasse 2, D-7750 Konstanz (DE)**

(72) Erfinder: **Eistetter, Klaus, Dr., Säntisblick 7, D-7750 Konstanz 19 (DE)**
Erfinder: **Kley, Hans-Peter, Dr., Alemannenstrasse 6, D-7753 Allensbach (DE)**
Erfinder: **Menge, Heinz-Günter, Dr., Ringstrasse 54, D-7750 Konstanz 19 (DE)**
Erfinder: **Schaefer, Hartmann, Dr., Zum Purren 27, D-7750 Konstanz 16 (DE)**

EP 0 023 706 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# 4-Phenoxypiperidine, Verfahren zu ihrer Herstellung, ihre Anwendung und sie enthaltende Arzneimittel

Die erfindungsgemäßen Verbindungen werden in der pharmazeutischen Industrie als Zwischenprodukte und zur Herstellung von Medikamenten verwendet.

In der DE-OS 2 735 051 werden 4-Phenylpiperidine beschrieben, die eine antidepressive, antiaggressive, diuretische, antiparkinsonwirksame, bronchodilatorische und antiarthritische Aktivität aufweisen. Bedeutung hinsichtlich der pharmakologischen Aktivität dieser Verbindungen wird einer Hydroxygruppe in 3-Stellung des Piperidinringes oder einem Esterderivat davon zugesprochen, besondere Bedeutung sollen veresterte Hydroxygruppen in 3- und 4-Stellung des Piperidinringes besitzen. Dagegen weisen nun bestimmte, in 3-Stellung unsubstituierte 4-Phenoxypiperidine pharmakologisch interessante und spezifische Eigenschaften auf.

Gegenstand der Erfindung sind 4-Phenoxypiperidine der allgemeinen Formel I

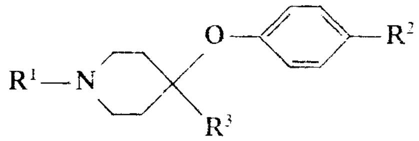

(I)

worin

$R^1$ ein Wasserstoffatom, eine Alkylgruppe mit 1 bis 5 Kohlenstoffatomen, eine Alkenylgruppe mit 3 bis 5 Kohlenstoffatomen, eine Cycloalkylmethylgruppe mit 3 bis 7 Kohlenstoffatomen im Cycloalkylteil oder eine Phenylalkylgruppe mit 1 bis 3 Kohlenstoffatomen im Alkylteil,

$R^2$ ein Wasserstoffatom, eine Nitrogruppe, eine Aminogruppe oder eine definitionsgemäße Acylaminogruppe und

$R^3$ eine Phenylgruppe oder eine Benzylgruppe

bedeuten, sowie ihre N-Oxide und ihre Säureadditionssalze.

Alkylgruppen und Alkenylgruppen können geradkettig oder verzweigt sein. Beispielsweise seien als Alkylgruppen die Methyl-, Ethyl-, Propyl-, Butyl-, Isopropyl-, Isobutyl-, sek.-Butyl-, tert.-Butyl-, Neopentyl- und tert.-Pentylgruppe und als Alkenylgruppe die Allyl- und 2-Methylallylgruppe genannt. Bevorzugt sind die Methyl-, tert.-Butyl- und Allylgruppe.

Als Cycloalkylmethylgruppe seien beispielsweise genannt die Cyclobutylmethyl-, Cycloheptylmethyl-, Cyclohexylmethyl-, Cyclopentylmethyl- und Cyclopropylmethylgruppe. Bevorzugt ist die Cyclopropylmethylgruppe.

Phenylalkylgruppen sind beispielsweise die Benzyl-, 1-Phenylethyl-, 2-Phenylethyl- und 3-Phenylpropylgruppe, bevorzugt ist die Benzylgruppe.

Acylreste von Acylaminogruppen sind Alkylcarbonylgruppen mit 1 bis 5 Kohlenstoffatomen im Alkylteil und Alkoxycarbonylgruppen mit 1 oder 2 Kohlenstoffatomen im Alkoxyteil und eine gegebenenfalls durch Chlor-, Brom-, Fluor-, Methyl-, Trifluormethyl- oder Methoxy-Substituenten substituierte Benzoylgruppe.

Als Salze kommen alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze werden durch dem Fachmann bekannte Verfahren in pharmakologisch verträgliche Salze übergeführt. Als solche seien beispielsweise genannt wasserlösliche oder wasserunlösliche Säureadditionssalze, wie das Hydrochlorid, Hydrobromid, Hydroiodid, Phosphat, Nitrat, Sulfat, Acetat, Citrat, Gluconat, Benzoat, Hibenzat [2-(4-Hydroxybenzoyl)-benzoat], Fendizoat (2-[(2'-Hydroxy-4-biphenylyl)-carbonyl]-benzoat), Propionat, Butyrat, Sulfosalicylat, Maleat, Laurat, Malat, Fumarat, Succinat, Oxalat, Tartrat, Amsonat (4,4'-Diaminostilben-2,2'-disulfonat), Embonat [4,4'-Methylen-bis-(3-hydroxy-2-naphthoat)], Metembonat [4,4'-Methylen-bis-(3-methoxy-2-naphthoat)], Stearat, Tosilat (p-Toluolsulfonat), 2-Hydroxy-3-naphthoat, 3-Hydroxy-2-naphthoat, Mesilat (Methansulfonat).

Eine Ausgestaltung der Erfindung sind 4-Phenoxypiperidine der allgemeinen Formel I*

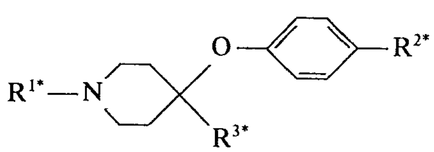

(I*)

worin

$R^{1*}$ ein Wasserstoffatom, eine geradkettige Alkylgruppe mit 1 bis 3 Kohlenstoffatomen oder eine Phenylalkylgruppe mit 1 oder 2 Kohlenstoffatomen im Alkylteil,

$R^{2*}$ ein Wasserstoffatom, eine Nitrogruppe, eine Aminogruppe oder eine Alkylcarbonylaminogruppe

mit 1 bis 5 Kohlenstoffatomen und

$R^{3*}$ eine Phenylgruppe oder eine Benzylgruppe

bedeuten, sowie ihre N-Oxide und ihre Säureadditionssalze.

Bevorzugte Vertreter der Ausgestaltung I* sind solche, in denen $R^{1*}$ ein Wasserstoffatom, eine Methylgruppe oder eine Benzylgruppe, $R^{2*}$ ein Wasserstoffatom, eine Nitrogruppe oder eine Aminogruppe und $R^{3*}$ eine Phenylgruppe oder eine Benzylgruppe bedeuten, sowie ihre N-Oxide und ihre Säureadditionssalze.

Besonders bevorzugte Vertreter der Ausgestaltung I* sind solche, in denen $R^{1*}$ ein Wasserstoffatom, eine Methylgruppe oder eine Benzylgruppe, $R^{2*}$ ein Wasserstoffatom, eine Nitrogruppe oder eine Aminogruppe und $R^{3*}$ eine Phenylgruppe bedeuten, sowie ihre pharmakologisch verträglichen Säureadditionssalze.

Ausgewählte Vertreter der Ausgestaltung I* sind solche, in denen $R^{1*}$ ein Wasserstoffatom, eine Methylgruppe oder eine Benzylgruppe, $R^{2*}$ eine Aminogruppe und $R^{3*}$ eine Phenylgruppe bedeuten, sowie ihre pharmakologisch verträglichen Säureadditionssalze.

Eine andere Ausgestaltung der Erfindung sind 4-Phenoxypiperidine der allgemeinen Formel I**

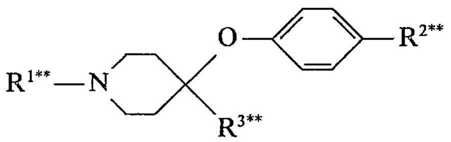

(I**)

worin

$R^{1**}$ eine verzweigtkettige Alkylgruppe mit 3 bis 5 Kohlenstoffatomen, eine Alkenylgruppe mit 3 bis 5 Kohlenstoffatomen oder eine Cycloalkylmethylgruppe mit 3 bis 5 Kohlenstoffatomen im Cycloalkylteil,

$R^{2**}$ ein Wasserstoffatom, eine Nitrogruppe, eine Aminogruppe oder eine Alkylcarbonylaminogruppe mit 1 bis 5 Kohlenstoffatomen und

$R^{3**}$ eine Phenylgruppe oder eine Benzylgruppe

bedeuten, sowie ihre N-Oxide und ihre Säureadditionssalze.

Bevorzugte Vertreter der Ausgestaltung I** sind solche, in denen $R^{1**}$ eine verzweigtkettige Alkylgruppe mit 3 bis 5 Kohlenstoffatomen, eine Allylgruppe oder eine Cyclopropylmethylgruppe, $R^{2**}$ ein Wasserstoffatom, eine Nitrogruppe oder eine Aminogruppe und $R^{3**}$ eine Phenylgruppe oder eine Benzylgruppe bedeuten, sowie ihre N-Oxide und ihre Säureadditionssalze.

Besonders bevorzugte Vertreter der Ausgestaltung I** sind solche, in denen $R^{1**}$ eine Isopropylgruppe oder tert.-Butylgruppe, $R^{2**}$ ein Wasserstoffatom, eine Nitrogruppe oder eine Aminogruppe und $R^{3**}$ eine Phenylgruppe bedeuten, sowie ihre pharmakologisch verträglichen Säureadditionssalze.

Als Vertreter der erfindungsgemäßen Verbindungen seien beispielsweise genannt

1-Ethyl-4-phenoxy-4-phenylpiperidin,
1-Cyclobutylmethyl-4-phenoxy-4-phenylpiperidin,
1-Isobutyl-4-phenoxy-4-phenylpiperidin,
1-(3-Phenylpropyl)-4-phenoxy-4-phenylpiperidin,
1-Neopentyl-4-phenoxy-4-phenylpiperidin,
1-Benzyl-4-phenoxy-4-phenylpiperidin,
1-Isopropyl-4-phenoxy-4-phenylpiperidin-N-oxid,
1-n-Butyl-4-benzyl-4-phenoxypiperidin,
4-Benzyl-1-ethyl-4-phenoxypiperidin,
4-Benzyl-1-cyclohexylmethyl-4-phenoxypiperidin,
4-Benzyl-1-propyl-4-phenoxypiperidin,
4-Benzyl-1-sek.-butyl-4-phenoxypiperidin,
4-Benzyl-1-isopentyl-4-phenoxypiperidin,
4-Benzyl-1-methyl-4-phenoxypiperidin-N-oxid,
1-Benzyl-4-(4-nitrophenoxy)-4-phenylpiperidin,
1-Cyclopentylmethyl-4-(4-nitrophenoxy)-4-phenylpiperidin,
1-Ethyl-4-(4-nitrophenoxy)-4-phenylpiperidin,
1-n-Butyl-4-(4-nitrophenoxy)-4-phenylpiperidin,
1-tert.-Butyl-4-(4-nitrophenoxy)-4-phenylpiperidin-N-oxid,
1-Neopentyl-4-(4-nitrophenoxy)-4-phenylpiperidin,
4-(4-Nitrophenoxy)-4-phenyl-1-(3-phenylpropyl)-piperidin,
1,4-Dibenzyl-4-phenoxypiperidin,
4-Benzyl-1-ethyl-4-phenoxypiperidin-N-oxid,

4-Benzyl-1-cyclopropylmethyl-4-phenoxypiperidin,
4-Benzyl-1-isobutyl-4-phenoxypiperidin,
4-Benzyl-1-isopentyl-4-phenoxypiperidin-N-oxid,
4-Benzyl-1-neopentyl-4-phenoxypiperidin,
4-Benzyl-1-n-butyl-4-phenoxypiperidin-N-oxid,
4-Benzyl-4-phenoxypiperidin,
4-(4-Aminophenoxy)-1-ethyl-4-phenylpiperidin,
4-(4-Aminophenoxy)-1-cyclopropylmethyl-4-phenylpiperidin,
4-(4-Aminophenoxy)-1-neopentyl-4-phenylpiperidin,
4-(4-Aminophenoxy)-4-phenyl-1-(3-phenylpropyl)-piperidin,
4-(4-Aminophenoxy)-1-n-butyl-4-phenylpiperidin,
1-Ethyl-4-(4-isobutyrylaminophenoxy)-4-phenylpiperidin,
1-Isopropyl-4-phenyl-4-(4-propionylaminophenoxy)piperidin,
1-n-Butyl-4-(4-n-hexanoylaminophenoxy)-4-phenylpiperidin,
4-Benzyl-1-ethyl-4-(4-isobutyrylaminophenoxy)piperidin,
4-Benzyl-1-isopropyl-4-(4-n-pentanoylaminophenoxy)piperidin,
4-Benzyl-1-ethyl-4-(4-propionylaminophenoxy)piperidin

und ihre Säureadditionssalze.

Ausgewählte erfindungsgemäß erhältliche Verbindungen sind

1-Methyl-4-phenoxy-4-phenylpiperidin,
4-(4-Aminophenoxy)-4-benzyl-1-methylpiperidin,
4-(4-Acetaminophenoxy)-1-methyl-4-phenylpiperidin, insbesondere
4-(4-Aminophenoxy)-1-methyl-4-phenylpiperidin

und ihre pharmakologisch verträglichen Säureadditionssalze.

Die 4-Phenoxypiperidine der allgemeinen Formel I bzw. der Ausgestaltungen I* und I**, ihre N-Oxide und ihre Säureadditionssalze weisen wertvolle Eigenschaften auf, die sie gewerblich verwertbar machen. Einerseits entfalten die Verbindungen, ihre N-Oxide und ihre pharmakologisch, d. h. biologisch verträglichen Säureadditionssalze eine ausgeprägte antiptotische Wirkung, die mit einer starken analgetischen Wirkung gekoppelt ist. Andererseits stellen die Verbindungen Zwischenprodukte zur Herstellung pharmakologisch wirksamer 4-Phenoxypiperidine dar.

So können z. B. Verbindungen der allgemeinen Formel I, in denen $R^1$ eine Phenylalkylgruppe, insbesondere eine Benzylgruppe bedeutet, durch Abspaltung der Phenylalkylgruppe in Verbindungen der Formel I übergeführt werden, in der $R^1$ ein Wasserstoffatom darstellt. Diese Verbindungen dienen als wertvolle Ausgangsverbindungen für die Herstellung von Derivaten der allgemeinen Formel I, in denen $R^1$ eine Alkyl-, Alkenyl- oder Cycloalkylmethylgruppe darstellt. 4-Phenoxypiperidine der allgemeinen Formel I, worin $R^2$ eine Nitrogruppe bedeutet, sind Zwischenprodukte für die Herstellung von solchen Verbindungen der allgemeinen Formel I, in der $R^2$ eine Amino- oder Acylaminogruppe oder ein Wasserstoffatom bedeutet.

Der antiptotische Effekt der erfindungsgemäßen Verbindungen findet seinen Ausdruck in einem starken Reserpinantagonismus bei niedriger Dosierung. Die ausgeprägte analgetische Wirkung läßt sich in verschiedenen Analgesie-Tiermodellen nachweisen, wobei diese Wirkung durch Naloxon antagonisierbar ist. Die zentralstimulierende Wirkung der erfindungsgemäßen Verbindungen wird durch Naloxon nicht antagonisiert, eher verstärkt, so daß auf verschiedene Wirkungszentren bei Säugetieren für die beiden Wirkungen geschlossen werden muß.

Die ausgezeichnete pharmakologische Wirksamkeit der erfindungsgemäßen Verbindungen ermöglicht ihren Einsatz in der Humanmedizin als Antidepressiva und Analgetica, wobei sie zur Prophylaxe und vor allem zur Behandlung bereits aufgetretener Symptome verwendet werden.

Als Indikationen für den humanmedizinischen Bereich seien beim Mann oder bei der Frau jeglichen Alters beispielsweise genannt Depressionen verschiedener Äthiologie und Symptomatologie, wie endogene Depressionen, psychogene Depressionen, Erschöpfungsdepressionen, depressive Verstimmungen; und Schmerzustände verschiedener Genese, insbesondere chronische Schmerzzustände, die zu Depressionen führen oder damit verbunden sind.

Ein weiterer Gegenstand der Erfindung sind daher die erfindungsgemäßen Verbindungen zur Anwendung bei der Bekämpfung der oben angegebenen Krankheiten. Ebenso umfaßt die Erfindung die Verwendung erfindungsgemäßer Verbindungen bei der Herstellung von Arzneimitteln, die zur Bekämpfung der angeführten Krankheiten eingesetzt werden.

Ein weiterer Gegenstand der Erfindung sind Arzneimittel, die ein oder mehrere 4-Phenoxypiperidine der allgemeinen Formel I

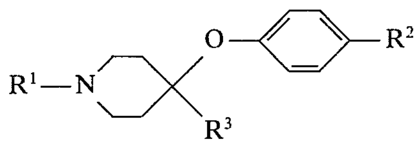

(I)

worin

$R^1$ ein Wasserstoffatom, eine Alkylgruppe mit 1 bis 5 Kohlenstoffatomen, eine Alkenylgruppe mit 3 bis 5 Kohlenstoffatomen, eine Cycloalkylmethylgruppe mit 3 bis 7 Kohlenstoffatomen im Cycloalkylteil oder eine Phenylalkylgruppe mit 1 bis 3 Kohlenstoffatomen im Alkylteil,
$R^2$ ein Wasserstoffatom, eine Nitrogruppe, eine Aminogruppe oder eine definitionsgemäße Acylaminogruppe und
$R^3$ eine Phenylgruppe oder eine Benzylgruppe

bedeuten, und/oder ihre pharmakologisch verträglichen N-Oxide und/oder ihre Säureadditionssalze enthalten.

Ausgestaltungen der Arzneimittel sind solche, die pharmakologisch aktive und verträgliche 4-Phenoxypiperidine der allgemeinen Formel I* oder I** oder ihre bevorzugten Vertreter, ihre N-Oxide und/oder ihre Säureadditionssalze enthalten.

Die erfindungsgemäßen Verbindungen werden nach an sich bekannten Verfahren zu Arzneimitteln formuliert. Als Arzneimittel können die neuen Verbindungen als solche oder gegebenenfalls in Kombination mit geeigneten pharmazeutischen Trägerstoffen eingesetzt werden. Enthalten die neuen pharmazeutischen Zubereitungen neben den Wirkstoffen pharmazeutische Trägerstoffe, beträgt der Wirkstoffgehalt dieser Mischungen 5 bis 95, vorzugsweise 25 bis 75 Gewichtsprozent der Gesamtmischung. Die Arzneimittel werden beispielsweise für die orale, rektale oder parenterale Gabe in geeigneten Dosen formuliert.

Die Tagesdosis für die orale Applikation an Menschen liegt im allgemeinen zwischen 1,0 bis 50, vorzugsweise 5 bis 50, insbesondere 10 bis 30 mg. Eine Einzelgabe enthält den oder die Wirkstoffe in Mengen von etwa 0,5 bis etwa 25, vorzugsweise 3 bis 15, insbesondere 5 bis 10 mg.

Die pharmazeutischen Zubereitungen bestehen in der Regel aus den erfindungsgemäßen Wirkstoffen und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzugs, eines Beutels oder eines anderen Behältnisses, für den therapeutisch aktiven Bestandteil in Anwendung kommen. Ein Trägerstoff kann z. B. als Vermittler für die Arzneimittelaufnahme durch den Körper, als Formulierungshilfsmittel, als Süßungsmittel, als Geschmackskorrigenz, als Farbstoff oder als Konservierungsmittel dienen.

Neben den erfindungsgemäßen 4-Phenoxypiperidinen, ihren pharmakologisch verträglichen N-Oxiden oder ihren Säureadditionssalzen können die pharmazeutischen Zubereitungen zusätzlich einen oder mehrere pharmakologisch aktive Bestandteile aus anderen Arzneimittelgruppen enthalten, beispielsweise milde Stimulantien, wie Coffein; Analgetika, wie Aminophenazon, Acetylsalicylsäure; Antiphlogistica, wie Phenylbutazon, Indometacin, (Hetero)arylessigsäuren; Minor-Tranquilizer, wie Meprobamat, Chlordiazepoxid, Diazepam; Major-Tranquilizer, wie Perazin, Fluophenazin, cerebraldurchblutungsfördernde und cerebralstoffwechselfördernde Mittel und/oder Roborantien, wie Glutaminsäure, anorganische Salze, Vitamine bzw. deren Kombinationen.

Das erfindungsgemäße Verfahren zur Herstellung der 4-Phenoxypiperidine der allgemeinen Formel I

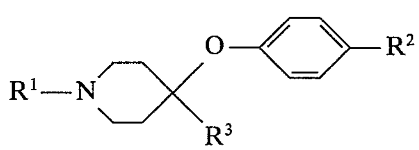

(I)

worin

$R^1$ ein Wasserstoffatom, eine Alkylgruppe mit 1 bis 5 Kohlenstoffatomen, eine Alkenylgruppe mit 3 bis 5 Kohlenstoffatomen, eine Cycloalkylmethylgruppe mit 3 bis 7 Kohlenstoffatomen im Cycloalkylteil oder eine Phenylalkylgruppe mit 1 bis 3 Kohlenstoffatomen im Alkylteil,
$R^2$ ein Wasserstoffatom, eine Nitrogruppe, eine Aminogruppe oder eine definitionsgemäße Acylaminogruppe und
$R^3$ eine Phenylgruppe oder eine Benzylgruppe

bedeuten, sowie ihrer N-Oxide und ihrer Säureadditionssalze, ist dadurch gekennzeichnet, daß man ein 4-Hydroxypiperidin der allgemeinen Formel II

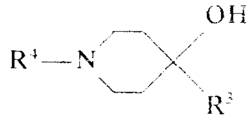

(II)

worin

$R^3$ die oben angegebene Bedeutung hat und

$R^4$ eine Alkylgruppe mit 1 bis 5 Kohlenstoffatomen, eine Alkenylgruppe mit 3 bis 5 Kohlenstoffatomen, eine Cycloalkylmethylgruppe mit 3 bis 7 Kohlenstoffatomen im Cycloalkylteil, eine Phenylalkylgruppe mit 1 bis 3 Kohlenstoffatomen im Alkylteil oder eine $R^5-CO$-Gruppe und

$R^5$ ein Wasserstoffatom, eine Alkylgruppe mit 1 bis 4 Kohlenstoffatomen, eine Alkenylgruppe mit 2 bis 4 Kohlenstoffatomen, eine Cycloalkylgruppe mit 3 bis 7 Kohlenstoffatomen, eine Phenylgruppe oder eine Phenylalkylgruppe mit 1 oder 2 Kohlenstoffatomen im Alkylteil

bedeuten, oder ein Salz davon mit 4-Nitrofluorbenzol in einem aprotischen Lösungsmittel in Gegenwart einer starken Base umsetzt und gegebenenfalls anschließend das erhaltene Reaktionsprodukt einem oder mehreren der folgenden Verfahrensschritte unterwirft.

a) Desalkylierung von erhaltenen Verbindungen der allgemeinen Formel I, worin $R^1$ eine definitionsgemäße Alkyl-, Alkenyl-, Cycloalkylmethyl- oder Phenalkylgruppe bedeutet, zu Verbindungen der allgemeinen Formel I, worin $R^1$ ein Wasserstoffatom darstellt;
b) Desacylierung von erhaltenen Verbindungen der allgemeinen Formel I, worin $R^1$ eine $R^5-CO$-Gruppe darstellt, zu Verbindungen der allgemeinen Formel I, worin $R^1$ ein Wasserstoffatom darstellt;
c) Alkylierung von erhaltenen Verbindungen der allgemeinen Formel I, worin $R^1$ ein Wasserstoffatom bedeutet, zu Verbindungen der allgemeinen Formel I, worin $R^1$ eine definitionsgemäße Alkyl-, Alkenyl-, Cycloalkylmethyl- oder Phenalkylgruppe bedeutet;
d) Reduktion von erhaltenen Verbindungen der allgemeinen Formel I, worin $R^1$ eine $R^5-CO$-Gruppe darstellt, zu Verbindungen der allgemeinen Formel I, worin $R^1$ eine definitionsgemäße Alkyl-, Alkenyl-, Cycloalkylmethyl- oder Phenalkylgruppe bedeutet;
e) Reduktion von erhaltenen Verbindungen der allgemeinen Formel I, worin $R^2$ eine Nitrogruppe darstellt, zu Verbindungen der allgemeinen Formel I, worin $R^2$ eine Aminogruppe darstellt, und gegebenenfalls anschließende Acylierung, wobei Acyl die definitionsgemäße Bedeutung hat;
f) Reduktion von erhaltenen Verbindungen der allgemeinen Formel I, worin $R^2$ eine Aminogruppe darstellt, zu Verbindungen der allgemeinen Formel I, worin $R^2$ ein Wasserstoffatom darstellt;
g) Umwandlung der erhaltenen Base in ein Säureadditionssalz oder eines erhaltenen Säureadditionssalzes in die Base;
h) Umwandlung der erhaltenen Base oder eines Säureadditionssalzes in das N-Oxid.

Die Umsetzung des Piperidinols II mit 4-Nitrofluorbenzol wird in aprotischen Lösungsmitteln, wie Dimethylformamid, Dimethylsulfoxid, Hexamethylphosphorsäuretriamid, bevorzugt in Dimethylformamid, durchgeführt. Als starke Basen seien beispielsweise Natriumhydrid, Kalium-tert.-butylat, Kalium-tert.-amylat, n-Butyllithium, Naphthalinnatrium, bevorzugt Natriumhydrid genannt. Das Piperidinol II wird dabei intermediär in das entsprechende Alkoholat bei Temperaturen zwischen 0 und 80°C, bevorzugt bei Raumtemperatur, übergeführt. Durch anschließende Zugabe von 4-Nitrofluorbenzol bei Temperaturen zwischen 0 und 100°C, bevorzugt 50 bis 60°C, erfolgt die Veretherung von II zum 4-(4-Nitrophenoxy)-piperidin I.

Die sich gegebenenfalls anschließenden Verfahrensschritte erfolgen nach an sich bekannten Methoden.

Die Desalkylierung, wobei Alkyl auch die definitionsgemäße Bedeutung von Alkenyl, Cycloalkylmethyl und Phenylalkyl, insbesondere Benzyl, einschließt, erfolgt beispielsweise mit Chlorameisensäureestern, wie Chlorameisensäureethylester, Chlorameisensäure-$\beta,\beta,\beta$-trichlorethylester ohne oder in Gegenwart inerter Lösungsmittel, wie Benzol, Toluol, Chloroform, bei erhöhter Temperatur, vorzugsweise bei Siedetemperatur des Lösungsmittels. Das erhaltene Zwischenprodukt wird mit wässerigen oder alkoholischen Lösungen von Basen, wie Natronlauge/Ethanol, Kalilauge/Butanol, bei erhöhter Temperatur, vorzugsweise der Siedetemperatur des Lösungsmittels, zum entsprechenden Desalkyl-4-phenoxypiperidin, d. h. zur Verbindung der allgemeinen Formel I, in der $R^1$ ein Wasserstoffatom bedeutet, umgesetzt.

Die Desalkylierung in der speziellen Form der Debenzylierung, d. h. bei Einsatz von erhaltenen Verbindungen der allgemeinen Formel I, worin $R^1$ Benzyl bedeutet, erfolgt alternativ durch Hydrogenolyse in Gegenwart von Katalysatoren, bevorzugt Palladium auf Kohle, in Lösungsmitteln, wie Methanol, Ethanol, Benzol, Cyclohexan, bei 0 bis 50°C, bevorzugt Raumtemperatur, und einem Wasserstoffdruck von 1–300 Atmosphären, bevorzugt 1 bis 5 Atmosphären.

Die Desacylierung von erhaltenen Verbindungen der allgemeinen Formel I, worin $R^1$ eine

$R^5-CO$-Gruppe bedeutet, erfolgt beispielsweise mit wässerigen oder alkoholischen Lösungen von Basen, wie Natronlauge/Ethanol, Kalilauge/Butanol, bei erhöhter Temperatur, vorzugsweise der Siedetemperatur des Lösungsmittels, zum entsprechenden Desacyl-4-phenoxypiperidin, d. h. zur Verbindung der allgemeinen Formel I, in der $R^1$ ein Wasserstoffatom bedeutet.

Die Alkylierung, wobei Alkyl auch die definitionsgemäße Bedeutung von Alkenyl, Phenalkyl und Cycloalkylmethyl einschließt, wird z. B. mit Alkylierungsmitteln $R^4-X$, worin $R^4$ die oben angegebene Bedeutung hat und X eine Fluchtgruppe bedeutet, wie Alkylhalogeniden, Alkylsulfonaten, z. B. -tosylaten, Alkylsulfaten, in inerten Lösungsmitteln, wie Aceton, Methylethylketon, Alkoholen, wie Methanol, Ethanol, Isopropanol, Dimethylformamid etc. oder ohne Lösungsmittel, unter Verwendung einer Hilfsbase, wie Natriumcarbonat, Kaliumcarbonat, Triethylamin, bei Temperaturen von etwa 20 bis 100°C durchgeführt. Die Alkylierung kann auch durch Umsetzung von erhaltenen Verbindungen der allgemeinen Formel I, worin $R^1$ ein Wasserstoffatom bedeutet, mit Acylderivaten $R^5-CO-X$, worin $R^5$ und X die oben angegebene Bedeutung haben, und anschließende Reduktion erfolgen. Geeignete Acylderivate sind beispielsweise Acetylchlorid, Propionylchlorid, Butyrylchlorid, Pivaloylchlorid, Cyclopropylcarbonylchlorid, Cyclobutylcarbonylchlorid, Benzoylchlorid, Phenylacetylchlorid.

Die Reduktion von erhaltenen Verbindungen der allgemeinen Formel I, worin $R^1$ eine $R^5-CO$-Gruppe darstellt und $R^5$ die oben angegebene Bedeutung hat, erfolgt z. B. durch Umsetzung mit einem komplexen Metallhydrid als Reduktionsmittel in einem wasserfreien organischen Lösungsmittel und hydrolytische Aufarbeitung. Geeignete Reduktionsmittel sind unter anderem Lithiumaluminiumhydrid (Lithiumhydridoaluminat) sowie Natriumdihydro-bis-(2-methoxyethoxy)-aluminat. Als Lösungsmittel sind inerte wasserfreie Ether, wie Diethylether, Tetrahydrofuran, Dioxan, 1,2-Dimethoxyethan und Diethylenglykoldiethylether, geeignet und ebenso aromatische Kohlenwasserstoffe, wie Benzol und Toluol, oder Gemische aus den genannten Verbindungen. Die Temperatur der Reaktion kann in weiten Grenzen, z. B. von 0 bis 100°C, schwanken. Gewöhnlich ist es am zweckmäßigsten, die Reaktion bei der Rückflußtemperatur des Reaktionsgemisches durchzuführen. Bei der bevorzugten Rückflußtemperatur ist die Reaktion normalerweise in 3 bis 4 Stunden beendet. Die Reaktionsteilnehmer können in äquivalenten Mengen verwendet werden, jedoch ist ein Überschuß des Reduktionsmittels bevorzugt. Anschließend an die Umsetzung mit dem Reduktionsmittel wird das Reaktionsprodukt durch Behandeln des Reaktionsgemisches mit einem wäßrigen Medium, wie Wasser, verdünnten wäßrigen anorganischen Säuren oder Basen oder anderen wasserhaltigen Medien, aufgearbeitet. Das Produkt kann durch Einstellen des pH-Wertes als freie Base oder als Säureadditionssalze isoliert werden.

Die Reduktion von erhaltenen Verbindungen der allgemeinen Formel I, worin $R^2$ eine Nitrogruppe darstellt, zu solchen, in denen $R^2$ eine Aminogruppe bedeutet, erfolgt z. B. durch Reaktion mit Wasserstoff in Gegenwart von Metallkatalysatoren, wie Raney-Nickel, Palladium, Platin, Platin oder Palladium auf Kohlenstoff, in geeigneten Lösungsmitteln, z. B. Alkanolen, unter Drücken von 1 bis 200 atm, bevorzugt bei Atmosphärendruck, oder durch Reduktion mit Hydrazin in Gegenwart von Raney-Nickel in Alkanolen, z. B. Ethanol (s. auch Houben-Weyl, Band 11/1, Seite 360 ff.). Eine sich gegebenenfalls anschließende Acylierung der Aminogruppe erfolgt durch Umsetzung mit entsprechenden Säureanhydriden oder -halogeniden (vgl. u. a. Houben-Weyl, Bd. 8, S. 655).

Die Reduktion der erhaltenen Verbindungen der allgemeinen Formel I, in denen $R^2$ eine Aminogruppe bedeutet, zu solchen, in denen $R^2$ ein Wasserstoffatom darstellt, erfolgt durch Diazotieren der Aminogruppe zur Diazoniumgruppe (vgl. Houben-Weyl, Band 10/3, S. 1 ff.) und deren anschließende Reduktion, z. B. mit Zink, bevorzugt unterphosphoriger Säure (vgl. Houben-Weyl, Band 10/3, S. 115 ff.).

Die erhaltenen Salze, z. B. die Hydrochloride, können durch Umsetzung mit wäßrigem Natrium- oder Kaliumhydroxid in die freie Base umgewandelt werden, die dann durch Lösungsmittelextraktion mit einem geeigneten, nicht mit Wasser mischbaren Lösungsmittel, wie Chloroform, Dichlormethan, Diethylether, Benzol, Toluol, Cyclohexan, gewonnen wird. Die freien Basen können auch durch Umsetzung eines Säureanlagerungssalzes mit Natriummethylat in Methanol und Isolierung der Base gewonnen werden. Salze können auch durch Überführung in die Base und weitere Umsetzung mit einer Säure in andere Salze, z. B. pharmakologisch verträgliche Säureadditionssalze übergeführt werden.

Säureanlagerungssalze erhält man durch Auflösen der 4-Phenoxypiperidine in einem geeigneten Lösungsmittel, z. B. Wasser, Aceton, einem niedermolekularen aliphatischen Alkohol (Ethanol, Isopropanol) oder Ether (Tetrahydrofuran, Diethylether), das die gewünschte Säure enthält, oder dem die gewünschte Säure anschließend zugegeben wird. Die Salze werden durch Filtrieren, Ausfällen mit einem Nichtlösungsmittel für das Säureadditionssalz oder durch Verdampfen des Lösungsmittels gewonnen.

Die N-Oxydation, d. h. die Überführung der erhaltenen Basen der allgemeinen Formel I oder ihrer Säureadditionssalze in die N-Oxide, wird vorzugsweise mit m-Chlorperbenzoesäure oder äquivalenten Oxydationsmitteln, wie Monoperschwefelsäure, Monoperphthalsäure, Peressigsäure, Trifluorperessigsäure, Perbenzoesäure, durchgeführt, wobei Temperaturen zwischen 0° und 80°C, zweckmäßigerweise um Raumtemperatur, eingehalten werden. Als Lösungsmittel werden die üblichen inerten Lösungsmittel, z. B. Benzol, Toluol, Chloroform, Methylenchlorid oder deren Gemische eingesetzt.

Die Ausgangsverbindungen der allgemeinen Formel II sind bekannt oder werden nach bekannten Verfahren hergestellt. Zur Herstellung der Verbindungen I* bzw. I** werden entsprechende Ausgangsverbindungen II* bzw. II** eingesetzt.

Die folgenden Beispiele dienen zur Erläuterung der Erfindung, ohne sie einzuschränken. Schmp. bedeutet Schmelzpunkt. Temperaturangaben erfolgen in °C

## Beispiele

### Beispiel 1

1-Methyl-4-(4-nitrophenoxy)-4-phenylpiperidin

Zu einer Suspension von 12,6 g Natriumhydrid (80%ig in Öl) in 200 ml absolutem Dimethylformamid tropft man unter Rühren und Feuchtigkeitsausschluß bei einer Temperatur von 45°C eine Lösung von 54 g 1-Methyl-4-phenyl-piperidin-4-ol in 200 ml absolutem Dimethylformamid. Hierbei tritt eine Wasserstoffentwicklung auf. Nach beendeter Zugabe rührt man noch 2 Stunden bei 50°C, läßt auf Raumtemperatur abkühlen und tropft anschließend eine Lösung von 49,4 g 4-Fluornitrobenzol in 100 ml absolutem Dimethylformamid zu. Man rührt noch 2 Stunden nach und gießt dann die Reaktionsmischung auf 800 ml Eiswasser. Der sich bildende Niederschlag wird abfiltriert und mit Wasser gewaschen. Umkristallisieren aus 250 ml Ethanol ergibt 52 g der Titelverbindung vom Schmp. 135–137°C.

### Beispiel 2

a) 4-Benzyl-1-methyl-4-(4-nitrophenoxy)-piperidin
Zu einer Suspension von 8,76 g Natriumhydrid (80%ig in Öl) in 200 ml absolutem Dimethylformamid tropft man unter Rühren und Feuchtigkeitsausschluß eine Lösung von 40,0 g 4-Benzyl-1-methyl-piperidin-4-ol in 120 ml absolutem Dimethylformamid. Hierbei tritt eine Wasserstoffentwicklung auf. Nach beendeter Zugabe rührt man 2 Stunden bei Raumtemperatur, tropft 31,6 g 4-Fluornitrobenzol zu und rührt 2 Stunden bei 50°C. Man gießt anschließend die braune Reaktionsmischung in 350 ml Eiswasser, rührt 30 Minuten und filtriert den abgeschiedenen Niederschlag ab, der mit Wasser und Petrolether gewaschen wird. Man löst das Rohprodukt in 150 ml siedendem Ethanol, dekantiert von ungelöstem Material, behandelt mit Aktivkohle und filtriert. Das abgekühlte Filtrat wird langsam mit 100 ml Petrolether versetzt, der gebildete Niederschlag abfiltriert und mit Petrolether gewaschen. Man erhält durch sukzessives Einengen des Filtrats 38,9 g der cremefarbenen Titelverbindung vom Schmp. 116–117°C.

Analog erhält man

b) 1-tert.-Butyl-4-(4-nitrophenoxy)-4-phenylpiperidin
aus 1-tert.-Butyl-4-phenyl-piperidin-4-ol und 4-Fluornitrobenzol, Schmp. 153–154°C.

c) 1-Isopropyl-4-(4-nitrophenoxy)-4-phenylpiperidin
aus 1-Isopropyl-4-phenylpiperidin-4-ol und 4-Fluornitrobenzol.

### Beispiel 3

a) 4-(4-Aminophenoxy)-1-methyl-4-phenylpiperidin
51 g 1-Methyl-4-(4-nitrophenoxy)-4-phenylpiperidin werden in 1,5 Liter Ethanol gelöst und bei einem Wasserstoffdruck von 1 Atmosphäre in Gegenwart von Platin hydriert. Man filtriert vom Katalysator ab, engt auf 300 ml ein und sammelt die abgeschiedenen Kristalle. Durch sekzessives Einengen des Filtrats erhält man 34,3 g der Titelverbindung vom Schmp. 173–174°C.

Analog erhält man durch Hydrierung der genannten Ausgangsverbindungen

b) 4-(4-Aminophenoxy)-1-tert.-butyl-4-phenylpiperidin
aus 1-tert.-Butyl-4-(4-nitrophenoxy)-4-phenylpiperidin

c) 4-(4-Aminophenoxy)-4-benzyl-1-methylpiperidin
[vom Schmp. 118–119°C (Essigester/Petrolether)] aus 4-Benzyl-1-methyl-4-(4-nitrophenoxy)-piperidin

d) 4-(4-Aminophenoxy)-4-phenyl-1-(2-phenylethyl)-piperidin aus 4-(4-Nitrophenoxy)-4-phenyl-1-(2-phenylethyl)-piperidin

Beispiel 4

a) 1-Methyl-4-phenoxy-4-phenylpiperidin
Zu einer Lösung von 10,0 g 4-(4-Aminophenoxy)-1-methyl-4-phenylpiperidin in 12 ml konzentrierter Salzsäure und 30 ml Wasser tropft man bei −10° C eine Lösung von 3,0 g Natriumnitrit in 18 ml Wasser. Man rührt 45 Minuten bei −5° C nach, tropft bei −5° C 50 ml 50%ige unterphosphorige Säure zu und läßt die Reaktionsmischung 24 Stunden bei 0° C stehen. Unter Eiskühlung wird mit 6n Natronlauge alkalisiert und mit Diethylether extrahiert. Nach dem Behandeln der organischen Phase mit Aktivkohle wird über Natriumsulfat getrocknet und eingeengt. Der Rückstand ergibt nach der Umkristallisation aus 100 ml n-Hexan 5,5 g der Titelverbindung vom Schmp. 99—101° C.

Analog erhält man

b) 4-Benzyl-1-methyl-4-phenoxypiperidin
[als Hydrochlorid vom Schmp. 197—198° C (aus Ethanol)] aus 4-(4-Aminophenoxy)-4-benzyl-1-methylpiperidin.

Beispiel 5

a) 4-Phenoxy-4-phenylpiperidin
9,0 g 1-Methyl-4-phenoxy-4-phenylpiperidin werden portionsweise zu 100 ml Chlorameisensäureethylester bei 80° C gegeben; hierbei wird starkes Aufschäumen beobachtet. Nach beendeter Zugabe kocht man 3 Stunden unter Rückfluß, destilliert im Vakuum den überschüssigen Chlorameisensäureethylester ab und versetzt den festen Rückstand mit 120 ml Wasser und 15 ml konzentriertem Ammoniak. Man rührt gut durch, filtriert den Niederschlag ab und wäscht mit Wasser nach. Das so erhaltene 1-Ethoxycarbonyl-4-phenoxy-4-phenylpiperidin (Schmp. 117—118° C aus Ethanol) wird mit einer Lösung von 15 g Kaliumhydroxid in 150 ml n-Butanol 4 Stunden unter Rückfluß gekocht; die Lösung wird nach dem Abkühlen mit 300 ml Wasser versetzt und zweimal mit Diethylether extrahiert. Die vereinigten organischen Phasen werden über Natriumsulfat getrocknet und eingeengt. Zurück bleibt die Titelverbindung als bräunliches Öl. Das Hydrochlorid schmilzt bei 192° C (aus Ethanol).

Analog erhält man

b) 4-(4-Nitrophenoxy)-4-phenylpiperidin (Öl)
aus 1-Methyl-4-(4-nitrophenoxy)-4-phenylpiperidin über 1-Ethoxycarbonyl-4-(4-nitrophenoxy)-4-phenylpiperidin (Schmp. 137—138° C).

Beispiel 6

a) 1-Isopropyl-4-(4-nitrophenoxy)-4-phenylpiperidin
6,0 g 4-(4-Nitrophenoxy)-4-phenylpiperidin, 2,8 g Kaliumcarbonat und 3,0 g Isopropylbromid werden in 50 ml Ethyl-methylketon 24 Stunden unter Rückfluß gekocht. Man dampft das Lösungsmittel ab, nimmt den Rückstand mit 100 ml Wasser auf und extrahiert mit 100 ml Diethylether. Die organische Phase wird über Natriumsulfat getrocknet, über Aktivkohle filtriert und eingeengt. Zurück bleiben 5,2 g der Titelverbindung als zähes, hellgelbes Öl.

Analog erhält man

b) 1-Allyl-4-(4-nitrophenoxy)-4-phenylpiperidin
aus 4-(4-Nitrophenoxy)-4-phenylpiperidin und Allylbromid

c) 4-(4-Nitrophenoxy)-4-phenyl-1-(2-phenylethyl)-piperidin
aus 4-(4-Nitrophenoxy)-4-phenylpiperidin und 2-Phenyl-ethylbromid.

Beispiel 7

a) 1-Cyclopropylmethyl-4-phenoxy-4-phenylpiperidin

1. Zu einer Lösung von 5,06 g 4-Phenoxy-4-phenylpiperidin und 2,5 g Triethylamin in 50 ml Dichlormethan tropft man bei 0–8°C 2,1 g Cyclopropancarbonsäurechlorid in 5 ml Methylenchlorid. Man rührt 2 Stunden bei 0°C, versetzt mit Wasser, trennt die organische Phase ab, extrahiert nochmals mit Dichlormethan, wäscht die vereinigten organischen Phasen mit verdünnter Salzsäure und Sodalösung, trocknet sie über Natriumsulfat und engt sie ein. Der ölige Rückstand (1-Cyclopropylcarbonyl-4-phenoxy-4-phenylpiperidin) wird in 50 ml Tetrahydrofuran gelöst, vorsichtig portionsweise mit 0,75 g Lithiumaluminiumhydrid versetzt und anschließend 1 Stunde unter Rückfluß gekocht. Nach dem Abkühlen wird zunächst tropfenweise 50 ml Wasser zugegeben und 2mal mit je 50 ml Diethylether extrahiert. Nach dem Trocknen der vereinigten organischen Phasen wird das Lösungsmittel abdestilliert. Zurück bleiben 4,6 g der Titelverbindung als hellgelbes zähes Öl.
2. Alternativ erhält man die Titelverbindung durch Alkylierung von 4-Phenoxy-4-phenylpiperidin mit Cyclopropylmethylbromid analog Beispiel 6a).

Analog erhält man

b) 1-(2,2-Dimethyl-1-propyl)-4-phenoxy-4-phenylpiperidin

1. aus 4-Phenoxy-4-phenylpiperidin und Pivaloylchlorid und anschließende Reduktion des Zwischenproduktes 4-Phenoxy-4-phenyl-1-pivaloylpiperidin mit Lithiumaluminiumhydrid oder alternativ
2. aus 4-Phenoxy-4-phenylpiperidin und Neopentylbromid analog Beispiel 6a).

Beispiel 8

a) 4-(4-Acetaminophenoxy)-1-methyl-4-phenylpiperidin

Zu einer Lösung von 2,8 g 4-(4-Aminophenoxy)-1-methyl-4-phenylpiperidin und 1,1 g Triethylamin in 10 ml Benzol tropft man eine Lösung von 0,85 g Acetylchlorid in 5 ml Benzol. Nach 6 Stunden Rühren bei Raumtemperatur engt man ein, nimmt mit je 30 ml Wasser und Diethylether auf, schüttelt gut durch, trennt die organische Phase ab, trocknet über Natriumsulfat und engt ein. Zurück bleiben 2,8 g der Titelverbindung als zähes gelbes Öl, das nach längerem Stehen aus Diethylether kristallisiert, Schmp. 143–144°C.

Analog erhält man

b) 1-Methyl-4-phenyl-4-(4-pivaloylaminophenoxy)-piperidin

aus 4-(4-Aminophenoxy)-1-methyl-4-phenylpiperidin und Pivaloylchlorid.

Beispiel 9

1-Methyl-4-phenoxy-4-phenylpiperidin-N-oxid

1,5 g 1-Methyl-4-phenoxy-4-phenylpiperidin werden in 4 ml Methanol gelöst und unter Eiskühlung mit 1,7 g 80%iger m-Chlorperoxybenzoesäure, gelöst in Methanol, langsam versetzt. Nach 3stündigem Rühren bei Raumtemperatur wird das Lösungsmittel abgezogen und der Rückstand zwischen konzentrierter Natronlauge und Chloroform verteilt. Das nach Trocknen der Chloroformphase und Verdampfen des Lösungsmittels verbleibende rohe N-Oxid-Hydrat wird in wenig Methanol aufgenommen und mit der äquivalenten Menge methanolischer Fumarsäure versetzt. Man erhält 1 g eines farblosen Niederschlags.

Die Verwendung von etherischer Salzsäure anstelle von methanolischer Fumarsäure ergibt das Hydrochlorid.

Beispiel 10

Tabletten

4-(4-Aminophenoxy)-1-methyl-4-phenylpiperidin (10 kg), Milchzucker (75 kg), Maisstärke (80 kg), hochdisperse Kieselsäure (3 kg) und Natriumlaurylsulfat (4 kg) werden gesiebt und gemischt. Polyvinylpyrrolidon (mittl. M.G. 25 000; 5 kg) wird in 20 l Wasser gelöst, und mit dieser Lösung wird die Pulvermischung gut befeuchtet. Die feuchte Mischung wird durch ein Sieb der Maschenweite 1,2 mm

granuliert. Nach dem Trocknen des Granulats werden Natriumcarboxymethylcellulose (16 kg), Talkum (5 kg) und Magnesiumstearat (2 kg) zugemischt. Die fertige Mischung wird zu Tabletten mit einem Durchmesser von 8 mm und einem Gewicht von 200 mg verpreßt.

Anstelle des 4-(4-Aminophenoxy)-1-methyl-4-phenylpiperidins kann auch eine andere erfindungsgemäße Verbindung, z. B. 1-Methyl-4-phenoxy-4-phenylpiperidin oder die Verbindungen in Form eines Salzes oder N-Oxids eingesetzt werden.

Beispiel 11

Kapseln

4-(4-Aminophenoxy)-1-methyl-4-phenylpiperidin (10 kg) und Milchzucker (sprühgetrocknet, 210 kg) werden gemischt und in Kapseln der Kapselgröße 3 abgefüllt.

Beispiel 12

Ampullen

1-Methyl-4-phenoxy-4-phenylpiperidin-hydrochlorid (2,500 kg) und Mannit (4,000 kg) werden in 80 Liter bidestilliertem Wasser gelöst und auf 100 Liter mit bidestilliertem Wasser aufgefüllt. Die Lösung wird durch ein Membranfilter mit einem Porendurchmesser von 0,1 μ filtriert, in 2-ml-Ampullen abgefüllt und 1 Stunde bei 100° C sterilisiert.

Pharmakologie

Die ausgeprägten antiptotischen und analgetischen Eigenschaften der erfindungsgemäßen Verbindungen lassen sich in mehreren Modellversuchen nachweisen, wobei sich die 4-Phenoxypiperidine infolge ihrer stärkeren Wirkung, der längeren Wirkungsdauer und der analgetischen Komponente dem handelsüblichen Antidepressivum Imipramin überlegen erweisen.

Der Vergleich der antiptotischen und analgetischen Eigenschaften der erfindungsgemäßen Verbindungen und der des Imipramins wurde am Beispiel der in Tabelle I angeführten Verbindungen durchgeführt.

Tabelle I

1. Imipramin
2. 1-Methyl-4-phenoxy-4-phenylpiperidin
3. 1-Methyl-4-(4-nitrophenoxy)-4-phenylpiperidin
4. 4-(4-Aminophenoxy)-4-benzyl-1-methylpiperidin
5. 4-(4-Aminophenoxy)-1-methyl-4-phenylpiperidin
6. 4-Benzyl-1-methyl-4-(4-nitrophenoxy)-piperidin
7. 4-Benzyl-1-methyl-4-phenoxypiperidin
8. 4-Phenoxy-4-phenylpiperidin
9. 1-tert.-Butyl-4-(4-nitrophenoxy)-4-phenylpiperidin
10. 4-(4-Acetaminophenoxy)-1-methyl-4-phenylpiperidin

In Tabelle II werden die ermittelten $DL_{50}$-Werte nach oraler Gabe sowie die in den verschiedenen Tests nach oraler Gabe erhaltenen $ED_{50}$-Werte (jeweils mg/kg) wiedergegeben.

Tabelle II

Antiptotische und analgetische Wirkung sowie akute Toxizität von 4-Phenoxypiperidinen im Vergleich mit Imipramin; $ED_{50}$- und $DL_{50}$-Werte nach oraler Applikation in [mg/kg] an der Maus
N: Naloxonantagonismus (+ total ⊕ mäßig ∅ nicht vorhanden)

| Lfd. Nr. | antiptotische Wirkung Reserpinantagonismus $ED_{50}$ (p.a.) 5 h | 6 h | 7 h | analgetische Wirkung Brennstrahltest $ED_{50}$ | N | Essigsäure-Writhingtest $ED_{50}$ | N | akute Toxizität $DL_{50}$ |
|---|---|---|---|---|---|---|---|---|
| 1 | 18 | 50 | 100 | >50 | | >100 | ∅ | 470 |
| 2 | 2.1 | 2.7 | 4.0 | 35 | + | | | 220 |
| 3 | 0.7 | 2.0 | 5.8 | 50 | | | | 140 |
| 4 | 9.5 | 24 | 50 | 2 | ⊕ | 27 | ⊕ | 180 |
| 5 | 0.7 | 3.0 | 10 | 12 | + | 7 | + | 160 |
| 6 | | | | >75 | | 35 | | 290 |
| 7 | | | | 55 | | 27 | | 300 |
| 8 | ~25 | >50 | >50 | 25 | | 50 | | 200 |
| 9 | | | | | | 10 | + | |
| 10 | <10 | <10 | <10 | 12,5 | | 10 | ⊕ | zwischen 100–200 |

Die Ermittlung der pharmakologischen Eigenschaften erfolgt nach folgenden Methoden:
Alle Experimente werden an weiblichen Albinomäusen NMRI, 20–30 g Körpergewicht, durchgeführt. 5 bis 20 Tiere/Dosis werden eingesetzt und Dosis-Wirkungs-Beziehungen ermittelt.

Antiptotische Wirkung
(Reserpinantagonismus)

Subcutane Applikation von 2 mg/kg Reserpin löst im Verlauf von mehreren Stunden bei Albinomäusen Ptosis aus [Reserpin-Ptosis, Domenjoz and Theobald (1959), Arch. Int. Pharmacodyn. 120, 450]. Die Tiere erhalten die Prüfsubstanzen in verschiedenen Dosen oral, 3 Stunden vor der Gabe von Reserpin. In der 2., 3. und 4. Stunde nach Reserpinapplikation (d. h. 5, 6 und 7 Stunden nach Substanzapplikation) wird der Grad der Ptosis nach einem Punktesystem 3–2–1–0 (volle, mäßige, leichte, keine Ptosis) festgestellt. Die geprüften Substanzen antagonisieren die Ptosis dosisabhängig. Es wird die $ED_{50}$ der antagonistischen Wirkung im Vergleich zur Reserpin-Tageskontrolle ausgewertet.

Analgesie

a) Brennstrahl-Test: Albinomäusen wird mit einem focussierten Wärmestrahl ein thermischer Schmerz an der Schwanzwurzel gesetzt und die Zeit bis zum Wegziehen des Schwanzes gestoppt. Normalerweise liegt sie im Bereich von 4 bis 5 Sekunden. Die geprüften Substanzen bewirken verzögerte Reaktion auf den Wärmeschmerz, d. h. eine verminderte Wärmeschmerzreaktion. Bestimmt wird die Dosis, die die Reaktionszeit um 50% verlängert. Literatur: D'Amour, F. E. and Smith D. L. (1941) J. Pharmacol. Exp. Ther. 72, 74.
b) Writhing-Test (Essigsäure-Writhing): Intraperitoneale Injektion von 0,2 ml/20 g Maus einer 0,75%igen Essigsäurelösung induziert bei Albinomäusen ein typisches, über den Körper mit Dorsalflektion ablaufendes Syndrom, »Writhing« genannt. Diese im Verlauf der ersten halben Stunde p. a. auftretenden »Writhings« werden im Zeitraum von 5 bis 20 Minuten nach Verabfolgung der Essigsäure gezählt. Die geprüften Substanzen (verabfolgt 30 Minuten vor

Essigsäure-Injektion) bewirken eine Verminderung der Anzahl der »Writhings«. Bestimmt wird die Dosis, die deren Anzahl, bezogen auf die Tageskontrolle, um 50% reduziert. Literatur: Koster, Anderson, de Beer (1959) Fed. Proc. 18, 412.

c) Naloxonapplikation (2 mg/kg sc.) 25 Minuten nach Gabe der Testsubstanzen vermag analgetische Wirkungen zu antagonisieren. Die Symbole +, ⊕, ∅ in der Tabelle, Spalte N, bedeuten totalen, mäßigen oder keinen Naloxonantagonismus.

Bestimmung der letalen Wirkung

Die Mäuse erhielten Futter (Altromin[R]) und Wasser ad libitum. Die zu prüfenden Substanzen wurden als Lösungen verschiedener Konzentration mit der Schlundsonde in einem Volumen von 10–20 ml/kg oral verabreicht; 5 Tiere pro Dosis wurden in Makrolonkäfigen, Typ II, gehalten. Die Beobachtungsdauer betrug 7 Tage. Die $DL_{50}$, die Dosis, bei der 50% der Tiere starben, wurde aus der Dosiswirkungskurve graphisch ermittelt. Literatur: Litchfield und Wilkoxon (1946) J. Pharm. Exp. Therap. 96/2, 99.

**Patentansprüche (DE, FR, IT, NL, BE, CH, GB, SE, LU)**

1. 4-Phenoxypiperidine der allgemeinen Formel I

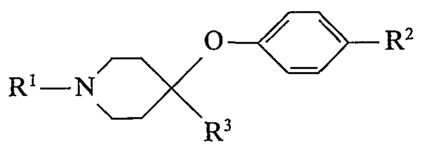

(I)

worin

$R^1$ ein Wasserstoffatom, eine Alkylgruppe mit 1 bis 5 Kohlenstoffatomen, eine Alkenylgruppe mit 3 bis 5 Kohlenstoffatomen, eine Cycloalkylmethylgruppe mit 3 bis 7 Kohlenstoffatomen im Cycloalkylteil oder eine Phenylalkylgruppe mit 1 bis 3 Kohlenstoffatomen im Alkylteil,

$R^2$ ein Wasserstoffatom, eine Nitrogruppe, eine Aminogruppe oder eine Acalaminogruppe, in der Acyl eine Alkylcarbonylgruppe mit 1 bis 5 Kohlenstoffatomen im Alkylteil oder eine Alkoxycarbonylgruppe mit 1 oder 2 Kohlenstoffatomen im Alkoxyteil oder eine gegebenenfalls durch Chlor-, Brom-, Fluor-, Methyl-, Trifluormethyl- oder Methoxy-Substituenten substituierte Benzoylgruppe darstellt, und

$R^3$ eine Phenylgruppe oder eine Benzylgruppe

bedeuten, sowie ihre N-Oxide und ihre Säureadditionssalze.

2. 4-Phenoxypiperidine nach Anspruch 1, gekennzeichnet durch die allgemeine Formel I*

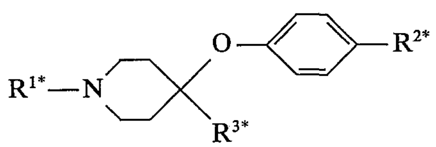

(I*)

worin

$R^{1*}$ ein Wasserstoffatom, eine geradkettige Alkylgruppe mit 1 bis 3 Kohlenstoffatomen oder eine Phenylalkylgruppe mit 1 oder 2 Kohlenstoffatomen im Alkylteil,

$R^{2*}$ ein Wasserstoffatom, eine Nitrogruppe, eine Aminogruppe oder eine Alkylcarbonylaminogruppe mit 1 bis 5 Kohlenstoffatomen und

$R^{3*}$ eine Phenylgruppe oder eine Benzylgruppe

bedeuten, sowie ihre N-Oxide und ihre Säureadditionssalze.

3. 4-Phenoxypiperidine nach Anspruch 2, in denen $R^{1*}$ ein Wasserstoffatom, eine Methylgruppe oder eine Benzylgruppe, $R^{2*}$ ein Wasserstoffatom, eine Nitrogruppe oder eine Aminogruppe und $R^{3*}$ eine Phenylgruppe oder eine Benzylgruppe bedeuten, sowie ihre N-Oxide und ihre Säureadditionssalze.

4. 4-Phenoxypiperidine nach Anspruch 2, in denen $R^{1*}$ ein Wasserstoffatom, eine Methylgruppe oder eine Benzylgruppe, $R^{2*}$ ein Wasserstoffatom, eine Nitrogruppe oder eine Aminogruppe und $R^{3*}$ eine Phenylgruppe bedeuten, sowie ihre pharmakologisch verträglichen Säureadditionssalze.

5. 4-Phenoxypiperidine nach Anspruch 2, in denen $R^{1*}$ ein Wasserstoffatom, eine Methylgruppe oder eine Benzylgruppe, $R^{2*}$ eine Aminogruppe und $R^{3*}$ eine Phenylgruppe bedeuten, sowie ihre pharmakologisch verträglichen Säureadditionssalze.

6. 4-Phenoxypiperidin nach Anspruch 2, in dem $R^{1*}$ eine Methylgruppe, $R^{2*}$ eine Aminogruppe und $R^{3*}$ eine Phenylgruppe bedeuten, sowie seine pharmakologisch verträglichen Säureadditionssalze.

7. 4-Phenoxypiperidine nach Anspruch 1, gekennzeichnet durch die allgemeine Formel I**

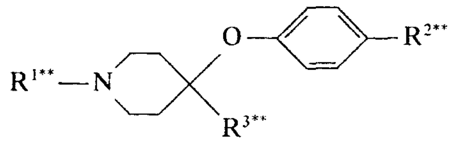

(I**)

worin

$R^{1**}$ eine verzweigtkettige Alkylgruppe mit 3 bis 5 Kohlenstoffatomen, eine Alkenylgruppe mit 3 bis 5 Kohlenstoffatomen oder eine Cycloalkylmethylgruppe mit 3 bis 5 Kohlenstoffatomen im Cycloalkylteil,
$R^{2**}$ ein Wasserstoffatom, eine Nitrogruppe, eine Aminogruppe oder eine Alkylcarbonylaminogruppe mit 1 bis 5 Kohlenstoffatomen und
$R^{3**}$ eine Phenylgruppe oder eine Benzylgruppe

bedeuten, sowie ihre N-Oxide und ihre Säureadditionssalze.

8. 4-Phenoxypiperidine nach Anspruch 7, in denen $R^{1**}$ eine verzweigtkettige Alkylgruppe mit 3 bis 5 Kohlenstoffatomen, eine Allylgruppe oder eine Cyclopropylmethylgruppe, $R^{2**}$ ein Wasserstoffatom, eine Nitrogruppe oder eine Aminogruppe und $R^{3**}$ eine Phenylgruppe oder eine Benzylgruppe bedeuten, sowie ihre N-Oxide und ihre Säureadditionssalze.

9. 4-Phenoxypiperidine nach Anspruch 7, in denen $R^{1**}$ eine Isopropylgruppe oder tert.-Butylgruppe, $R^{2**}$ ein Wasserstoffatom, eine Nitrogruppe oder eine Aminogruppe und $R^{3**}$ eine Phenylgruppe bedeuten, sowie ihre pharmakologisch verträglichen Säureadditionssalze.

10. Verbindungen gemäß einem der Ansprüche 1 bis 9 zur Anwendung bei der Bekämpfung von Depressionen und Schmerzzuständen.

11. Arzneimittel, enthaltend eine pharmakologisch verträgliche Verbindung nach einem der Ansprüche 1 bis 9.

12. Verfahren zur Herstellung der 4-Phenoxypiperidine der allgemeinen Formel I

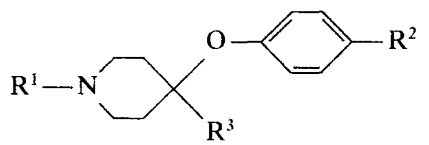

(I)

worin

$R^1$ ein Wasserstoffatom, eine Alkylgruppe mit 1 bis 5 Kohlenstoffatomen, eine Alkenylgruppe mit 3 bis 5 Kohlenstoffatomen, eine Cycloalkylmethylgruppe mit 3 bis 7 Kohlenstoffatomen im Cycloalkylteil oder eine Phenylalkylgruppe mit 1 bis 3 Kohlenstoffatomen im Alkylteil,
$R^2$ ein Wasserstoffatom, eine Nitrogruppe, eine Aminogruppe oder eine Acylaminogruppe, wobei Acyl die in Anspruch 1 angegebene Bedeutung hat, und
$R^3$ eine Phenylgruppe oder eine Benzylgruppe

bedeuten, sowie ihrer N-Oxide und ihrer Säureadditionssalze, dadurch gekennzeichnet, daß man ein 4-Hydroxypiperidin der allgemeinen Formel II

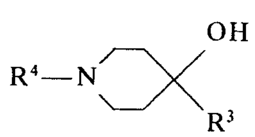

(II)

worin

$R^3$ die oben angegebene Bedeutung hat und
$R^4$ eine Alkylgruppe mit 1 bis 5 Kohlenstoffatomen, eine Alkenylgruppe mit 3 bis 5 Kohlenstoffatomen, eine Cycloalkylmethylgruppe mit 3 bis 7 Kohlenstoffatomen im Cycloalkylteil, eine Phenylalkylgruppe mit 1 bis 3 Kohlenstoffatomen im Alkylteil oder eine $R^5-CO$-Gruppe und
$R^5$ ein Wasserstoffatom, eine Alkylgruppe mit 1 bis 4 Kohlenstoffatomen, eine Alkenylgruppe mit 2 bis 4 Kohlenstoffatomen, eine Cycloalkylgruppe mit 3 bis 7 Kohlenstoffatomen, eine Phenylgruppe oder eine Phenylalkylgruppe mit 1 oder 2 Kohlenstoffatomen im Alkylteil

bedeuten, oder ein Salz davon mit 4-Nitrofluorbenzol in einem aprotischen Lösungsmittel in

Gegenwart einer starken Base umsetzt und gegebenenfalls anschließend das erhaltene Reaktionsprodukt einem oder mehreren der folgenden Verfahrensschritte unterwirft:

a) Desalkylierung von erhaltenen Verbindungen der allgemeinen Formel I, worin $R^1$ eine definitionsgemäße Alkyl-, Alkenyl-, Cycloalkylmethyl- oder Phenalkylgruppe bedeutet, zu Verbindungen der allgemeinen Formel I, worin $R^1$ ein Wasserstoffatom darstellt;
b) Desacylierung von erhaltenen Verbindungen der allgemeinen Formel I, worin $R^1$ eine $R^5-CO$-Gruppe darstellt, zu Verbindungen der allgemeinen Formel I, worin $R^1$ ein Wasserstoffatom darstellt;
c) Alkylierung von erhaltenen Verbindungen der allgemeinen Formel I, worin $R^1$ ein Wasserstoffatom bedeutet, zu Verbindungen der allgemeinen Formel I, worin $R^1$ eine definitionsgemäße Alkyl-, Alkenyl-, Cycloalkylmethyl- oder Phenalkylgruppe bedeutet;
d) Reduktion von erhaltenen Verbindungen der allgemeinen Formel I, worin $R^1$ eine $R^5-CO$-Gruppe darstellt, zu Verbindungen der allgemeinen Formel I, worin $R^1$ eine definitionsgemäße Alkyl-, Alkenyl-, Cycloalkylmethyl- oder Phenalkylgruppe bedeutet;
e) Reduktion von erhaltenen Verbindungen der allgemeinen Formel I, worin $R^2$ eine Nitrogruppe darstellt, zu Verbindungen der allgemeinen Formel I, worin $R^2$ eine Aminogruppe darstellt, und gegebenenfalls anschließende Acylierung, wobei Acyl die definitionsgemäße Bedeutung hat;
f) Reduktion von erhaltenen Verbindungen der allgemeinen Formel I, worin $R^2$ eine Aminogruppe darstellt, zu Verbindungen der allgemeinen Formel I, worin $R^2$ ein Wasserstoffatom darstellt;
g) Umwandlung der erhaltenen Base in ein Säureadditionssalz oder eines erhaltenen Säureadditionssalzes in die Base;
h) Umwandlung der erhaltenen Base oder eines Säureadditionssalzes in das N-Oxid.

**Patentansprüche (AT)**

1. Verfahren zur Herstellung von 4-Phenoxypiperidinen der allgemeinen Formel I

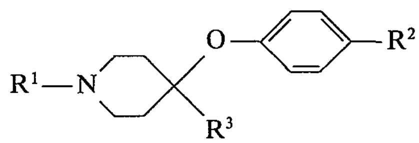

(I)

worin

$R^1$ ein Wasserstoffatom, eine Alkylgruppe mit 1 bis 5 Kohlenstoffatomen, eine Alkenylgruppe mit 3 bis 5 Kohlenstoffatomen, eine Cycloalkylmethylgruppe mit 3 bis 7 Kohlenstoffatomen im Cycloalkylteil oder eine Phenylalkylgruppe mit 1 bis 3 Kohlenstoffatomen im Alkylteil,
$R^2$ ein Wasserstoffatom, eine Nitrogruppe, eine Aminogruppe oder eine Acylaminogruppe, in der Acyl eine Alkylcarbonylgruppe mit 1 bis 5 Kohlenstoffatomen im Alkylteil oder eine Alkoxycarbonylgruppe mit 1 oder 2 Kohlenstoffatomen im Alkoxyteil oder eine gegebenenfalls durch Chlor-, Brom-, Fluor-, Methyl-, Trifluormethyl- oder Methoxy-Substituenten substituierte Benzoylgruppe darstellt, und
$R^3$ eine Phenylgruppe oder eine Benzylgruppe

bedeuten, sowie ihrer N-Oxide und ihrer Säureadditionssalze, dadurch gekennzeichnet, daß man ein 4-Hydroxypiperidin der allgemeinen Formel II

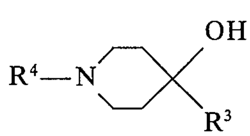

(II)

worin

$R^3$ die oben angegebene Bedeutung hat und
$R^4$ eine Alkylgruppe mit 1 bis 5 Kohlenstoffatomen, eine Alkenylgruppe mit 3 bis 5 Kohlenstoffatomen, eine Cycloalkylmethylgruppe mit 3 bis 7 Kohlenstoffatomen im Cycloalkylteil, eine Phenylalkylgruppe mit 1 bis 3 Kohlenstoffatomen im Alkylteil oder eine $R^5-CO$-Gruppe und
$R^5$ ein Wasserstoffatom, eine Alkylgruppe mit 1 bis 4 Kohlenstoffatomen, eine Alkenylgruppe mit 2 bis 4 Kohlenstoffatomen, eine Cycloalkylgruppe mit 3 bis 7 Kohlenstoffatomen, eine Phenylgruppe oder eine Phenylalkylgruppe mit 1 oder 2 Kohlenstoffatomen im Alkylteil

bedeuten, oder ein Salz davon mit 4-Nitrofluorbenzol in einem aprotischen Lösungsmittel in

Gegenwart einer starken Base umsetzt und gegebenenfalls anschließend das erhaltene Reaktionsprodukt einem oder mehreren der folgenden Verfahrensschritte unterwirft:

a) Desalkylierung von erhaltenen Verbindungen der allgemeinen Formel I, worin $R^1$ eine definitionsgemäße Alkyl-, Alkenyl-, Cycloalkylmethyl- oder Phenalkylgruppe bedeutet, zu Verbindungen der allgemeinen Formel I, worin $R^1$ ein Wasserstoffatom darstellt;
b) Desacylierung von erhaltenen Verbindungen der allgemeinen Formel I, worin $R^1$ eine $R^5-CO$-Gruppe darstellt, zu Verbindungen der allgemeinen Formel I, worin $R^1$ ein Wasserstoffatom darstellt;
c) Alkylierung von erhaltenen Verbindungen der allgemeinen Formel I, worin $R^1$ ein Wasserstoffatom bedeutet, zu Verbindungen der allgemeinen Formel I, worin $R^1$ eine definitionsgemäße Alkyl-, Alkenyl-, Cycloalkylmethyl- oder Phenalkylgruppe bedeutet;
d) Reduktion von erhaltenen Verbindungen der allgemeinen Formel I, worin $R^1$ eine $R^5-CO$-Gruppe darstellt, zu Verbindungen der allgemeinen Formel I, worin $R^1$ eine definitionsgemäße Alkyl-, Alkenyl-, Cycloalkylmethyl- oder Phenalkylgruppe bedeutet;
e) Reduktion von erhaltenen Verbindungen der allgemeinen Formel I, worin $R^2$ eine Nitrogruppe darstellt, zu Verbindungen der allgemeinen Formel I, worin $R^2$ eine Aminogruppe darstellt, und gegebenenfalls anschließende Acylierung, wobei Acyl die definitionsgemäße Bedeutung hat;
f) Reduktion von erhaltenen Verbindungen der allgemeinen Formel I, worin $R^2$ eine Aminogruppe darstellt, zu Verbindungen der allgemeinen Formel I, worin $R^2$ ein Wasserstoffatom darstellt;
g) Umwandlung der erhaltenen Base in ein Säureadditionssalz oder eines erhaltenen Säureadditionssalzes in die Base;
h) Umwandlung der erhaltenen Base oder eines Säureadditionssalzes in das N-Oxid.

2. Verfahren nach Anspruch 1, dadurch gekennzeichnet, daß man 4-Phenoxypiperidine der allgemeinen Formel I*

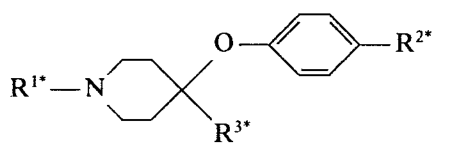

(I*)

worin

$R^{1*}$ ein Wasserstoffatom, eine geradkettige Alkylgruppe mit 1 bis 3 Kohlenstoffatomen oder eine Phenylalkylgruppe mit 1 oder 2 Kohlenstoffatomen im Alkylteil,
$R^{2*}$ ein Wasserstoffatom, eine Nitrogruppe, eine Aminogruppe oder eine Alkylcarbonylaminogruppe mit 1 bis 5 Kohlenstoffatomen und
$R^{3*}$ eine Phenylgruppe oder eine Benzylgruppe

bedeuten, sowie ihre N-Oxide und ihre Säureadditionssalze durch Umsetzung eines 4-Hydroxypiperidins der allgemeinen Formel II*

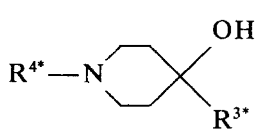

(II*)

worin

$R^{3*}$ die oben angegebene Bedeutung hat und
$R^{4*}$ eine Alkylgruppe mit 1 bis 3 Kohlenstoffatomen, eine Phenylalkylgruppe mit 1 oder 2 Kohlenstoffatomen im Alkylteil oder eine $R^{5*}-CO$-Gruppe und
$R^{5*}$ ein Wasserstoffatom, eine Alkylgruppe mit 1 oder 2 Kohlenstoffatomen, eine Phenylgruppe oder eine Benzylgruppe

bedeuten, oder eines Salzes davon mit 4-Nitrofluorbenzol in einem aprotischen Lösungsmittel in Gegenwart einer starken Base herstellt und gegebenenfalls anschließend das erhaltene Reaktionsprodukt einem oder mehreren der folgenden Verfahrensschritte unterwirft:

a) Desalkylierung von erhaltenen Verbindungen der allgemeinen Formel I*, worin $R^{1*}$ eine oben angegebene Alkyl- oder Phenalkylgruppe bedeutet, zu Verbindungen der allgemeinen Formel I*, worin $R^{1*}$ ein Wasserstoffatom darstellt;
b) Desacylierung von erhaltenen Verbindungen der allgemeinen Formel I*, worin $R^{1*}$ eine

$R^{5*}$—CO-Gruppe darstellt, zu Verbindungen der allgemeinen Formel I*, worin $R^{1*}$ ein Wasserstoffatom darstellt;

c) Alkylierung von erhaltenen Verbindungen der allgemeinen Formel I*, worin $R^{1*}$ ein Wasserstoffatom bedeutet, zu Verbindungen der allgemeinen Formel I*, worin $R^{1*}$ eine oben angegebene Alkyl- oder Phenalkylgruppe bedeutet;

d) Reduktion von erhaltenen Verbindungen der allgemeinen Formel I*, worin $R^{1*}$ eine $R^{5*}$—CO-Gruppe darstellt, zu Verbindungen der allgemeinen Formel I*, worin $R^{1*}$ eine oben angegebene Alkyl- oder Phenalkylgruppe bedeutet;

e) Reduktion von erhaltenen Verbindungen der allgemeinen Formel I*, worin $R^{2*}$ eine Nitrogruppe darstellt, zu Verbindungen der allgemeinen Formel I*, worin $R^{2*}$ eine Aminogruppe darstellt, und gegebenenfalls anschließende Acylierung, wobei Acyl die oben angegebene Bedeutung von Alkylcarbonyl hat;

f) Reduktion von erhaltenen Verbindungen der allgemeinen Formel I*, worin $R^{2*}$ eine Aminogruppe darstellt, zu Verbindungen der allgemeinen Formel I*, worin $R^{2*}$ ein Wasserstoffatom darstellt;

g) Umwandlung der erhaltenen Base in ein Säureadditionssalz oder eines erhaltenen Säureadditionssalzes in die Base;

h) Umwandlung der erhaltenen Base oder eines Säureadditionssalzes in das N-Oxid.

3. Verfahren nach Anspruch 2, dadurch gekennzeichnet, daß man 4-Phenoxypiperidine der Formel I*, in denen $R^{1*}$ ein Wasserstoffatom, eine Methylgruppe oder eine Benzylgruppe, $R^{2*}$ ein Wasserstoffatom, eine Nitrogruppe oder eine Aminogruppe und $R^{3*}$ eine Phenylgruppe oder eine Benzylgruppe bedeuten, ihre N-Oxide oder ihre Säureadditionssalze herstellt.

4. Verfahren nach Anspruch 2, dadurch gekennzeichnet, daß man 4-Phenoxypiperidine der Formel I*, in denen $R^{1*}$ ein Wasserstoffatom, eine Methylgruppe oder eine Benzylgruppe, $R^{2*}$ ein Wasserstoffatom, eine Nitrogruppe oder eine Aminogruppe und $R^{3*}$ eine Phenylgruppe bedeuten, oder ihre pharmakologisch verträglichen Säureadditionssalze herstellt.

5. Verfahren nach Anspruch 2, dadurch gekennzeichnet, daß man 4-Phenoxypiperidine der Formel I*, in denen $R^{1*}$ ein Wasserstoffatom, eine Methylgruppe oder eine Benzylgruppe, $R^{2*}$ eine Aminogruppe und $R^{3*}$ eine Phenylgruppe bedeuten, oder ihre pharmakologisch verträglichen Säureadditionssalze herstellt.

6. Verfahren nach Anspruch 2, dadurch gekennzeichnet, daß man das 4-Phenoxypiperidin der Formel I*, worin $R^{1*}$ eine Methylgruppe, $R^{2*}$ eine Aminogruppe und $R^{3*}$ eine Phenylgruppe bedeuten, oder seine pharmakologisch verträglichen Säureadditionssalze herstellt.

7. Verfahren nach Anspruch 1, dadurch gekennzeichnet, daß man 4-Phenoxypiperidine der allgemeinen Formel I**

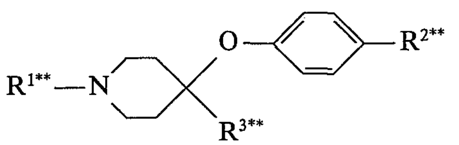

(I**)

worin

$R^{1**}$ eine verzweigtkettige Alkylgruppe mit 3 bis 5 Kohlenstoffatomen, eine Alkenylgruppe mit 3 bis 5 Kohlenstoffatomen oder eine Cycloalkylmethylgruppe mit 3 bis 5 Kohlenstoffatomen im Cycloalkylteil,

$R^{2**}$ ein Wasserstoffatom, eine Nitrogruppe, eine Aminogruppe oder eine Alkylcarbonylaminogruppe mit 1 bis 5 Kohlenstoffatomen und

$R^{3**}$ eine Phenylgruppe oder eine Benzylgruppe

bedeuten, sowie ihre N-Oxide und ihre Säureadditionssalze durch Umsetzung eines 4-Hydroxypiperidins der allgemeinen Formel II**

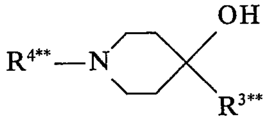

(II**)

worin

$R^{3**}$ die oben angegebene Bedeutung hat und

$R^{4**}$ eine verzweigtkettige Alkylgruppe mit 3 bis 5 Kohlenstoffatomen, eine Alkenylgruppe mit 3 bis 5 Kohlenstoffatomen, eine Cycloalkylmethylgruppe mit 3 bis 5 Kohlenstoffatomen im Cycloalkylteil oder eine $R^{5**}$—CO-Gruppe und

$R^{5'''}$ eine Alkylgruppe mit 2 bis 4 Kohlenstoffatomen, eine Alkenylgruppe mit 2 bis 4 Kohlenstoffatomen oder eine Cycloalkylgruppe mit 3 bis 5 Kohlenstoffatomen

bedeuten, oder eines Salzes davon mit 4-Nitrofluorbenzol in einem aprotischen Lösungsmittel in Gegenwart einer starken Base herstellt und gegebenenfalls anschließend das erhaltene Reaktionsprodukt einem oder mehreren der folgenden Verfahrensschritte unterwirft:

a) Desalkylierung von erhaltenen Verbindungen der allgemeinen Formel I**, worin $R^{1'''}$ eine oben angegebene Alkyl-, Alkenyl- oder Cycloalkylmethylgruppe bedeutet, zu Verbindungen der allgemeinen Formel I**, worin $R^{1'''}$ ein Wasserstoffatom darstellt;
b) Desacylierung von erhaltenen Verbindungen der allgemeinen Formel I**, worin $R^{1'''}$ eine $R^{5'''}$—CO-Gruppe darstellt, zu Verbindungen der allgemeinen Formel I**, worin $R^{1'''}$ ein Wasserstoffatom darstellt;
c) Alkylierung von erhaltenen Verbindungen der allgemeinen Formel I**, worin $R^{1'''}$ ein Wasserstoffatom bedeutet, zu Verbindungen der allgemeinen Formel I**, worin $R^{1'''}$ eine oben angegebene Alkyl-, Alkenyl- oder Cycloalkylmethylgruppe bedeutet;
d) Reduktion von erhaltenen Verbindungen der allgemeinen Formel I**, worin $R^{1'''}$ eine $R^{5'''}$—CO-Gruppe darstellt, zu Verbindungen der allgemeinen Formel I**, worin $R^{1'''}$ eine oben angegebene Alkyl-, Alkenyl- oder Cycloalkylmethylgruppe bedeutet;
e) Reduktion von erhaltenen Verbindungen der allgemeinen Formel I**, worin $R^{2'''}$ eine Nitrogruppe darstellt, zu Verbindungen der allgemeinen Formel I**, worin $R^{2'''}$ eine Aminogruppe darstellt, und gegebenenfalls anschließende Acylierung, wobei Acyl die oben angegebene Bedeutung von Alkylcarbonyl hat;
f) Reduktion von erhaltenen Verbindungen der allgemeinen Formel I**, worin $R^{2'''}$ eine Aminogruppe darstellt, zu Verbindungen der allgemeinen Formel I**, worin $R^{2'''}$ ein Wasserstoffatom darstellt;
g) Umwandlung der erhaltenen Base in ein Säureadditionssalz oder eines erhaltenen Säureadditionssalzes in die Base;
h) Umwandlung der erhaltenen Base oder eines Säureadditionssalzes in das N-Oxid.

8. Verfahren nach Anspruch 7, dadurch gekennzeichnet, daß man 4-Phenoxypiperidine der allgemeinen Formel I**, in denen $R^{1'''}$ eine verzweigtkettige Alkylgruppe mit 3 bis 5 Kohlenstoffatomen, eine Allylgruppe oder eine Cyclopropylmethylgruppe, $R^{2'''}$ ein Wasserstoffatom, eine Nitrogruppe oder eine Aminogruppe und $R^{3'''}$ eine Phenylgruppe oder eine Benzylgruppe bedeuten, ihre N-Oxide oder ihre Säureadditionssalze herstellt.

9. Verfahren nach Anspruch 7, dadurch gekennzeichnet, daß man 4-Phenoxypiperidine der allgemeinen Formel I**, in denen $R^{1'''}$ eine Isopropylgruppe oder tert.-Butylgruppe, $R^{2'''}$ ein Wasserstoffatom, eine Nitrogruppe oder eine Aminogruppe und $R^{3'''}$ eine Phenylgruppe bedeuten, oder ihre pharmakologisch verträglichen Säureadditionssalze herstellt.

**Claims for the contracting states DE, FR, IT, NL, BE, CH, GB, SE, LU**

1. 4-Phenoxypiperidines of the general formula I

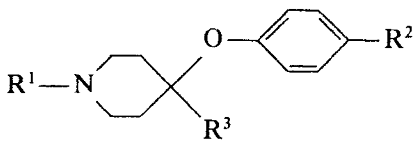

(I)

wherein

$R^1$ denotes a hydrogen atom, an alkyl group with 1 to 5 carbon atoms, an alkenyl group with 3 to 5 carbon atoms, a cycloalkylmethyl group with 3 to 7 carbon atoms in the cycloalkyl part or a phenylalkyl group with 1 to 3 carbon atoms in the alkyl part,
$R^2$ denotes a hydrogen atom, a nitro group, an amino group or an acylamino group in which acyl represents an alkylcarbonyl group with 1 to 5 carbon atoms in the alkyl part or an alkoxycarbonyl group with 1 or 2 carbon atoms in the alkoxy part or a benzoyl group optionally substituted by chloro, bromo, fluoro, methyl, trifluoromethyl or methoxy substituents, and
$R^3$ denotes a phenyl group or a benzyl group,

and their N-oxides and their acid addition salts.

2. 4-Phenoxypiperidines according to Claim 1, characterised by the general formula I*

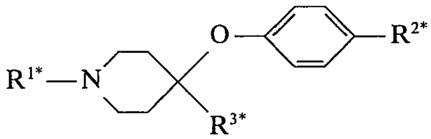

(I*)

wherein

$R^{1*}$ denotes a hydrogen atom, a straight-chain alkyl group with 1 to 3 carbon atoms or a phenylalkyl group with 1 or 2 carbon atoms in the alkyl part,
$R^{2*}$ denotes a hydrogen atom, a nitro group, an amino group or an alkylcarbonylamino group with 1 to 5 carbon atoms in the alkyl part,
$R^{3*}$ denotes a phenyl group or a benzyl group,

and their N-oxides and their acid addition salts.

3. 4-Phenoxypiperidines according to Claim 2, in which $R^{1*}$ denotes a hydrogen atom, a methyl group or a benzyl group, $R^{2*}$ denotes a hydrogen atom, a nitro group or an amino group and $R^{3*}$ denotes a phenyl group or a benzyl group, and their N-oxides and their acid addition salts.

4. 4-Phenoxypiperidines according to Claim 2, in which $R^{1*}$ denotes a hydrogen atom, a methyl group or a benzyl group, $R^{2*}$ denotes a hydrogen atom, a nitro group or an amino group and $R^{3*}$ denotes a phenyl group, and their pharmacologically acceptable acid addition salts.

5. 4-Phenoxypiperidines according to Claim 2, in which $R^{1*}$ denotes a hydrogen atom, a methyl group or a benzyl group, $R^{2*}$ denotes an amino group and $R^{3*}$ denotes a phenyl group, and their pharmacologically acceptable acid addition salts.

6. 4-Phenoxypiperidine according to Claim 2, in which $R^{1*}$ denotes a methyl group, $R^{2*}$ denotes an amino group and $R^{3*}$ denotes a phenyl group, and its pharmacologically acceptable acid addition salts.

7. 4-Phenoxypiperidines according to Claim 1, characterised by the general formula I**

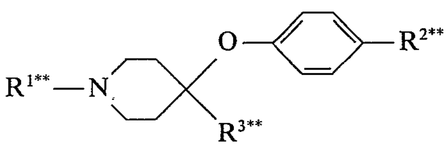

(I**)

wherein

$R^{1**}$ denotes a branched alkyl group with 3 to 5 carbon atoms, an alkenyl group with 3 to 5 carbon atoms or a cycloalkylmethyl group with 3 to 5 carbon atoms in the cycloalkyl part,
$R^{2**}$ denotes a hydrogen atom, a nitro group, an amino group or an alkylcarbonylamino group with 1 to 5 carbon atoms in the alkyl part, and
$R^{3**}$ denotes a phenyl group or a benzyl group,

and their N-oxides and their acid addition salts.

8. 4-Phenoxypiperidines according to Claim 7, in which $R^{1**}$ denotes a branched alkyl group with 3 to 5 carbon atoms, an allyl group or a cyclopropylmethyl group, $R^{2**}$ denotes a hydrogen atom, a nitro group or an amino group and $R^{3**}$ denotes a phenyl group or a benzyl group, and their N-oxides and their acid addition salts.

9. 4-Phenoxypiperidines according to Claim 7, in which $R^{1**}$ denotes an isopropyl group or a tert.-butyl group, $R^{2**}$ denotes a hydrogen atom, a nitro group or an amino group and $R^{3**}$ denotes a phenyl group, and their pharmacologically acceptable acid addition salts.

10. Compounds according to anyone of Claims 1 to 9 for use in combating depressions and painful conditions.

11. Medicaments containing a pharmacologically acceptable compound according to anyone of Claims 1 to 9.

12. Process for the preparation of the 4-phenoxypiperidines of the general formula I

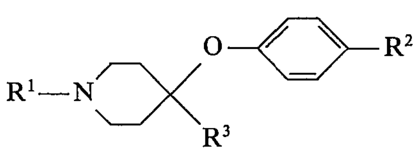

(I)

wherein

$R^1$ denotes a hydrogen atom, an alkyl group with 1 to 5 carbon atoms, an alkenyl group with 3 to 5 carbon atoms, a cycloalkylmethyl group with 3 to 7 carbon atoms in the cycloalkyl part or a phenylalkyl group with 1 to 3 carbon atoms in the alkyl part,

$R^2$ denotes a hydrogen atom, a nitro group, an amino group or an acylamino group, in which acyl has the meaning indicated in Claim 1, and
$R^3$ denotes a phenyl group or a benzyl group,

and their N-oxides and their acid addition salts, characterised in that a 4-hydroxypiperidine of the general formula II

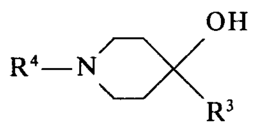

(II)

wherein

$R^3$ has the meaning indicated above and
$R^4$ denotes an alkyl group with 1 to 5 carbon atoms, an alkenyl group with 3 to 5 carbon atoms, a cycloalkylmethyl group with 3 to 7 carbon atoms in the cycloalkyl part, a phenylalkyl group with 1 to 3 carbon atoms in the alkyl part or an $R^5-CO$ group and
$R^5$ denotes a hydrogen atom, an alkyl group with 1 to 4 carbon atoms, an alkenyl group with 2 to 4 carbon atoms, a cycloalkyl group with 3 to 7 carbon atoms, a phenyl group or a phenylalkyl group with 1 or 2 carbon atoms in the alkyl part,

or a salt thereof, is reacted with 4-nitrofluorobenzene in an aprotic solvent in the presence of a strong base and the resulting reaction product is then optionally subjected to one or more of the following process steps:

a) dealkylation of resulting compounds of the general formula I wherein $R^1$ denotes an alkyl, alkenyl, cycloalkylmethyl or phenalkyl group as herein defined, to give compounds of the general formula I wherein $R^1$ represents a hydrogen atom;
b) deacylation of resulting compunds of the general formula I wherein $R^1$ represents an $R^5-CO$ group to give compounds of the general formula I wherein $R^1$ represents a hydrogen atom;
c) alkylation of the resulting compounds of the general formula I wherein $R^1$ denotes a hydrogen atom to give compounds of the general formula I wherein $R^1$ denotes an alkyl, alkenyl, cycloalkylmethyl or phenalkyl group as herein defined;
d) reduction of resulting compounds of the general formula I wherein $R^1$ represents an $R^5-CO$ group to give compounds of the general formula I wherein $R^1$ denotes an alkyl, alkenyl, cycloalkylmethyl or phenalkyl group as herein defined;
e) reduction of resulting compounds of the general formula I wherein $R^2$ represents a nitro group to give compounds of the general formula I wherein $R^2$ represents an amino group, and optionally, subsequent acylation, wherein acyl has the meaning as herein defined;
f) reduction of resulting compounds of the general formula I wherein $R^2$ represents an amino group to give compounds of the general formula I wherein $R^2$ represents a hydrogen atom;
g) conversion of the resulting base into an acid addition salt or of a resulting acid addition salt into the base; and
h) conversion of the resulting base or of an acid addition salt into the N-oxide.

**Claims for the contracting state AT**

1. Process for the preparation of 4-phenoxypiperidines of the general formula I

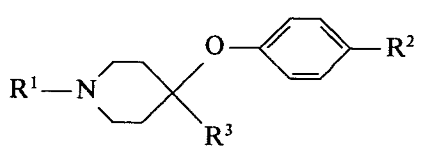

(I)

wherein

$R^1$ denotes a hydrogen atom, an alkyl group with 1 to 5 carbon atoms, an alkenyl group with 3 to 5 carbon atoms, a cycloalkylmethyl group with 3 to 7 carbon atoms in the cycloalkyl part or a phenylalkyl group with 1 to 3 carbon atoms in the alkyl part,
$R^2$ denotes a hydrogen atom, a nitro group, an amino group or an acylamino group in which acyl represents an alkylcarbonyl group with 1 to 5 carbon atoms in the alkyl part or an alkoxycarbonyl group with 1 or 2 carbon atoms in the alkoxy part or a benzoyl group optionally substituted by

chloro, bromo, fluoro, methyl, trifluoromethyl or methoxy substituents, and
$R^3$ denotes a phenyl group or a benzyl group,

and their N-oxides and their acid addition salts, characterised in that a 4-hydroxypiperidine of the general formula II

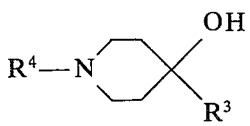

(II)

wherein

$R^3$ has the meaning indicated above and
$R^4$ denotes an alkyl group with 1 to 5 carbon atoms, an alkenyl group with 3 to 5 carbon atoms, a cycloalkylmethyl group with 3 to 7 carbon atoms in the cycloalkyl part, a phenylalkyl group with 1 to 3 carbon atoms in the alkyl part or an $R^5-CO$ group and
$R^5$ denotes a hydrogen atom, an alkyl group with 1 to 4 carbon atoms, an alkenyl group with 2 to 4 carbon atoms, a cycloalkyl group with 3 to 7 carbon atoms, a phenyl group or a phenylalkyl group with 1 or 2 carbon atoms in the alkyl part,

or a salt thereof, is reacted with 4-nitrofluorobenzene in an aprotic solvent in the presence of a strong base and the resulting reaction product is then optionally subjected to one or more of the following process steps:

a) dealkylation of resulting compounds of the general formula I wherein $R^1$ denotes an alkyl, alkenyl, cycloalkylmethyl or phenalkyl group as herein defined, to give compounds of the general formula I wherein $R^1$ represents a hydrogen atom;
b) deacylation of resulting compounds of the general formula I wherein $R^1$ represents an $R^5-CO$ group to give compounds of the general formula I wherein $R^1$ represents a hydrogen atom;
c) alkylation of the resulting compounds of the general formula I wherein $R^1$ denotes a hydrogen atom to give compounds of the general formula I wherein $R^1$ denotes an alkyl, alkenyl, cycloalkylmethyl or phenalkyl group as herein defined;
d) reduction of resulting compounds of the general formula I wherein $R^1$ represents an $R^5-CO$ group to give compounds of the general formula I wherein $R^1$ denotes an alkyl, alkenyl, cycloalkylmethyl or phenalkyl group as herein defined;
e) reduction of resulting compounds of the general formula I wherein $R^2$ represents a nitro group to give compounds of the general formula I wherein $R^2$ represents an amino group, and optionally, subsequent acylation, wherein acyl has the meaning as herein defined;
f) reduction of resulting compounds of the general formula I wherein $R^2$ represents an amino group to give compounds of the general formula I wherein $R^2$ represents a hydrogen atom;
g) conversion of the resulting base into an acid addition salt or of a resulting acid addition salt into the base; and
h) conversion of the resulting base or of an acid addition salt into the N-oxide.

2. Process according to Claim 1, characterised in that 4-phenoxypiperidines of the general formula I*

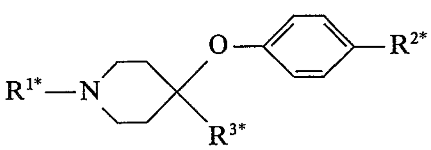

(I*)

wherein

$R^{1*}$ denotes a hydrogen atom, a straight-chain alkyl group with 1 to 3 carbon atoms or a phenyl-alkyl group with 1 or 2 carbon atoms in the alkyl part,
$R^{2*}$ denotes a hydrogen atom, a nitro group, an amino group or an alkylcarbonylamino group with 1 to 5 carbon atoms in the alkyl part,
$R^{3*}$ denotes a phenyl group or a benzyl group,

and their N-oxides and their acid addition salts are prepared by reaction of a 4-hydroxypiperidine of the general formula II*

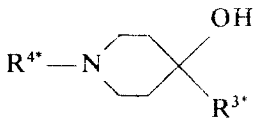

(II*)

wherein

$R^{3*}$ has the meaning indicated above and
$R^{4*}$ denotes an alkyl group with 1 to 3 carbon atoms, a phenylalkyl group with 1 or 2 carbon atoms in the alkyl part or an $R^{5*}-CO$ group and
$R^{5*}$ denotes a hydrogen atom, an alkyl group with 1 or 2 carbon atoms, a phenyl group or a benzyl group,

or a salt thereof, with 4-nitrofluorobenzene in an aprotic solvent in the presence of a strong base and the resulting reaction product is then optionally subjected to one or more of the following process steps:

a) dealkylation of resulting compounds of the general formula I* wherein $R^{1*}$ denotes an alkyl or phenalkyl group as indicated above, to give compounds of the general formula I* wherein $R^{1*}$ represents a hydrogen atom;
b) deacylation of resulting compounds of the general formula I* wherein $R^{1*}$ represents an $R^{5*}-CO$ group to give compounds of the general formula I* wherein $R^{1*}$ represents a hydrogen atom;
c) alkylation of resulting compounds of the general formula I* wherein $R^{1*}$ denotes a hydrogen atom to give compounds of the general formula I* wherein $R^{1*}$ denotes an alkyl or phenalkyl group as indicated above;
d) reduction of resulting compounds of the general formula I* wherein $R^{1*}$ represents an $R^{5*}-CO$ group to give compounds of the general formula I* wherein $R^{1*}$ denotes an alkyl or phenalkyl group as indicated above;
e) reduction of resulting compounds of the general formula I* wherein $R^{2*}$ represents a nitro group to give compounds of the general formula I* wherein $R^{2*}$ represents an amino group, and optionally, subsequent acylation, wherein acyl has the meaning as indicated above;
f) reduction of resulting compounds of the general formula I* wherein $R^{2*}$ represents an amino group to give compounds of the general formula I* wherein $R^{2*}$ represents a hydrogen atom;
g) conversion of the resulting base into an acid addition salt or of a resulting acid addition salt into the base; and
h) conversion of the resulting base or of an acid addition salt into the N-oxide.

3. Process according to Claim 2, characterised in that 4-phenoxypiperidines of the formula I* in which $R^{1*}$ denotes a hydrogen atom, a methyl group or a benzyl group, $R^{2*}$ denotes a hydrogen atom, a nitro group or an amino group and $R^{3*}$ denotes a phenyl group or a benzyl group, their N-oxides and their acid addition salts are prepared.

4 Process according to Claim 2, characterised in that 4-phenoxypiperidines of the formula I* in which $R^{1*}$ denotes a hydrogen atom, a methyl group or a benzyl group, $R^{2*}$ denotes a hydrogen atom, a nitro group or an amino group and $R^{3*}$ denotes a phenyl group, or their pharmacologically acceptable acid addition salts are prepared.

5. Process according to Claim 2, characterised in that 4-phenoxypiperidines of the formula I* in which $R^{1*}$ denotes a hydrogen atom, a methyl group or a benzyl group, $R^{2*}$ denotes an amino group and $R^{3*}$ denotes a phenyl group, or their pharmacologically acceptable acid addition salts are prepared.

6. Process according to Claim 2, characterised in that the 4-phenoxypiperidine of the formula I* in which $R^{1*}$ denotes a methyl group, $R^{2*}$ denotes an amino group and $R^{3*}$ denotes a phenyl group, or its pharmacologically acceptable acid addition salts are prepared.

7. Process according to Claim 1, characterised in that 4-phenoxypiperidines of the general formula I**

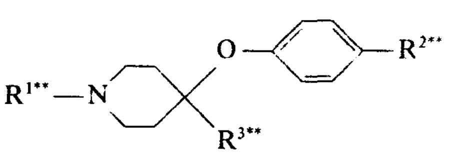

(I**)

wherein

$R^{1**}$ denotes a branched alkyl group with 3 to 5 carbon atoms, an alkenyl group with 3 to 5 carbon atoms or a cycloalkylmethyl group with 3 to 5 carbon atoms in the cycloalkyl part,
$R^{2**}$ denotes a hydrogen atom, a nitro group, an amino group or an alkylcarbonylamino group with 1 to 5 carbon atoms in the alkyl part, and
$R^{3**}$ denotes a phenyl group or a benzyl group,

and their N-oxides and their acid addition salts, are prepared by reaction of a 4-hydroxypiperidine of the general formula II**

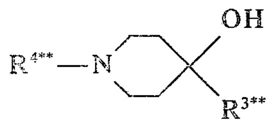

(II**)

wherein

$R^{3**}$ has the meaning indicated above and

$R^{4**}$ denotes a branched alkyl group with 3 to 5 carbon atoms, an alkenyl group with 3 to 5 carbon atoms, a cycloalkylmethyl group with 3 to 5 carbon atoms in the cycloalkyl part or an $R^{5**}-CO$ group and

$R^{5**}$ denotes an alkyl group with 2 to 4 carbon atoms, an alkenyl group with 2 to 4 carbon atoms or a cycloalkyl group with 3 to 5 carbon atoms,

or a salt thereof, with 4-nitrofluorobenzene in an aprotic solvent in the presence of a strong base and the resulting reaction product is then optionally subjected to one or more of the following-process steps:

a) dealkylation of resulting compounds of the general formula I**, wherein $R^{1**}$ denotes an alkyl, alkenyl or cycloalkylmethyl group as indicated above, to give compounds of the general formula I** wherein $R^{1**}$ represents a hydrogen atom;
b) deacylation of resulting compounds of the general formula I** wherein $R^{1**}$ represents an $R^{5**}-CO$ group to give compounds of the general formula I** wherein $R^{1**}$ represents a hydrogen atom;
c) alkylation of the resulting compounds of the general formula I** wherein $R^{1**}$ denotes a hydrogen atom to give compounds of the general formula I** wherein $R^{1**}$ denotes an alkyl, alkenyl or cycloalkylmethyl group as indicated above;
d) reduction of resulting compounds of the general formula I** wherein $R^{1**}$ represents an $R^{5**}-CO$ group to give compounds of the general formula I** wherein $R^{1**}$ denotes an alkyl, alkenyl or cycloalkylmethyl group as indicated above;
e) reduction of resulting compounds of the general formula I** wherein $R^{2**}$ represents a nitro group to give compounds of the general formula I** wherein $R^{2**}$ represents an amino group, and optionally, subsequent acylation wherein acyl has the meaning of alkylcarbonyl indicated above;
f) reduction of resulting compounds of the general formula I** wherein $R^{2**}$ represents an amino group to give compounds of the general formula I** wherein $R^{2**}$ represents a hydrogen atom;
g) conversion of the resulting base into an acid addition salt or of a resulting acid addition salt into the base; and
h) conversion of the resulting base or of an acid addition salt into the N-oxide.

8. Process according to Claim 7, characterised in that 4-phenoxypiperidines of the general formula I** in which $R^{1**}$ denotes a branched alkyl group with 3 to 5 carbon atoms, an allyl group or a cyclopropylmethyl group, $R^{2**}$ denotes a hydrogen atom, a nitro group or an amino group and $R^{3**}$ denotes a phenyl group or a benzyl group, or their acid addition salts are prepared.

9. Process according to Claim 7, characterised in that 4-phenoxypiperidines of the general formula I** in which $R^{1**}$ denotes an isopropyl group or a tert.-butyl group, $R^{2**}$ denotes a hydrogen atom, a nitro group or an amino group and $R^{3**}$ denotes a phenyl group, or their pharmacologically acceptable acid addition salts are prepared.

**Revendications pour les Etats contractant DE, FR, IT, NL, BE, CH, GB, SE, LU**

1. 4-phénoxypipéridines de formule générale I ci-dessous:

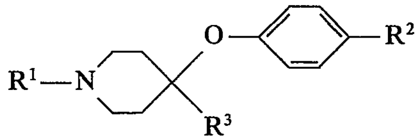

(I)

dans laquelle:

$R^1$ représente un atome d'hydrogène, un groupe alkyle comprenant de 1 à 5 atomes de carbone, un groupe alcényle comprenant de 3 à 5 atomes de carbone, un groupe cycloalkylméthyle

comprenant de 3 à 7 atomes de carbone dans la partie cycloalkyle ou un groupe phénylalkyle comprenant de 1 à 3 atomes de carbone dans la partie alkyle,

$R^2$ représente un atome d'hydrogène, un groupe nitro-, un groupe amino- ou un groupe acylamino- dans lequel l»acyle« représente un groupe alkylcarbonyle comportant de 1 à 5 atomes de carbone dans la partie alkyle, un groupe alcoxycarbonyle comportant 1 ou 2 atomes de carbone dans la partie alcoxy, ou un groupe benzoyle éventuellement substitué par des atomes de chlore, de brome, de fluor, des groupes méthyle, trifluorméthyle ou méthoxy-, et

$R^3$ représente un groupe phényle ou benzyle,

ainsi que leurs N-oxydes et leurs sels d'addition d'acides.

2. 4-phénoxypipéridines selon la revendication 1, caractérisés par la formule générale I* ci-dessous:

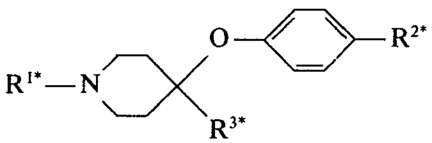

(I*)

dans laquelle:

$R^{1*}$ représente un atome d'hydrogène, un groupe alkyle à chaîne droite comportant de 1 à 3 atomes de carbone, ou un groupe phénylalkyle comportant 1 ou 2 atomes de carbone dans la partie alkyle,

$R^{2*}$ représente un atome d'hydrogène, un groupe nitro-, un groupe amino- ou un groupe alkylcarbonylamino- comportant de 1 à 5 atomes de carbone, et

$R^{3*}$ représente un groupe phényle ou benzyle,

ainsi que leurs N-oxydes et leurs sels d'addition d'acides.

3. 4-phénoxypipéridines selon la revendication 2, dans lesquelles $R^{1*}$ est un atome d'hydrogène, un groupe méthyle ou benzyle, $R^{2*}$ est un atome d'hydrogène, un groupe nitro- ou un groupe amino- et $R^{3*}$ est un groupe phényle ou benzyle, ainsi que leurs N-oxydes et leurs sels d'addition d'acides.

4. 4-phénoxypipéridines selon la revendication 2, dans lesquelles $R^{1*}$ est un atome d'hydrogène, un groupe méthyle ou benzyle, $R^{2*}$ est un atome d'hydrogène, un groupe nitro- ou un groupe amino- et $R^{3*}$ est un groupe phényle, ainsi que leurs sels d'addition d'acides pharmacologiquement compatibles.

5. 4-phénoxypipéridines selon la revendication 2, dans lesquelles $R^{1*}$ est un atome d'hydrogène, un groupe méthyle ou un groupe benzyle, $R^{2*}$ est un groupe amino- et $R^{3*}$ est un groupe phényle, ainsi que leurs sels d'addition d'acides pharmacologiquement compatibles.

6. 4-phénoxypipéridine selon la revendication 2, dans laquelle $R^{1*}$ est un groupe méthyle, $R^{2*}$ est un groupe amino- et $R^{3*}$ est un groupe phényle, ainsi que ses sels d'addition d'acides pharmacologiquement compatibles.

7. 4-phénoxypipéridines selon la revendication 1, caractérisées par la formule générale I** ci-dessous:

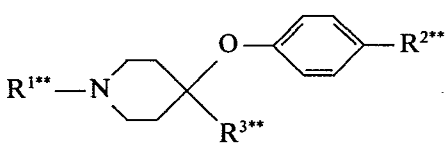

(I**)

dans laquelle:

$R^{1**}$ représente un groupe alkyle à chaîne ramifiée comportant de 3 à 5 atomes de carbone, un groupe alcényle comportant de 3 à 5 atomes de carbone ou un groupe cycloalkylméthyle comportant de 3 à 5 atomes de carbone dans la partie cycloalkyle,

$R^{2**}$ représente un atome d'hydrogène, un groupe nitro-, un groupe amino- ou un groupe alkylcarbonylamino- comportant de 1 à 5 atomes de carbone, et,

$R^{3**}$ représente un groupe phényle ou benzyle,

ainsi que leurs N-oxydes et leurs sels d'addition d'acides.

8. 4-phénoxypipéridines selon la revendication 7, dans lesquelles $R^{1**}$ est un groupe alkyle à chaîne ramifiée comportant de 3 à 5 atomes de carbone, un groupe allyle ou cyclopropylméthyle, $R^{2**}$ est un atome d'hydrogène, un groupe nitro- ou un groupe amino- et $R^{3**}$ est un groupe phényle ou benzyle, ainsi que leurs N-oxydes et leurs sels d'addition d'acides.

9. 4-phénoxypipéridines selon la revendication 7, dans lesquelles $R^{1**}$ est un groupe isopropyle ou butyle tertiaire, $R^{2**}$ est un atome d'hydrogène, un groupe nitro- ou un groupe amino- et $R^{3**}$ est un groupe phényle, ainsi que leurs sels d'addition d'acides pharmacologiquement compatibles.

10. Composés selon l'une des revendications 1 à 9, pour l'utilisation pour le traitement des dépressions et des états douloureux.

11. Médicaments contenant un composé pharmacologiquement compatible, selon l'une des revendications 1 à 9.

12. Procédé pour la préparation des 4-phénoxypipéridines de formule générale I ci-après:

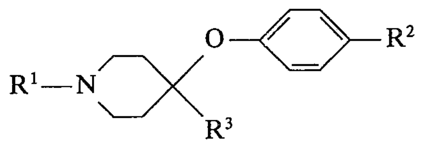

(I)

dans laquelle:

$R^1$ représente un atome d'hydrogène, un groupe alkyle comportant de 1 à 5 atomes de carbone, un groupe alcényle comportant de 3 à 5 atomes de carbone, un groupe cycloalkylméthyle comportant de 3 à 7 atomes de carbone dans la partie cycloalkyle ou un groupe phénylalkyle comportant de 1 à 3 atomes de carbone dans la partie alkyle,

$R^2$ représente un atome d'hydrogène, un groupe nitro-, un groupe amino- ou un groupe acylamino-, »acyl« ayant la signification indiquée dans la revendication 1 et,

$R^3$ représente un groupe phényle ou benzyle,

ainsi que de leurs N-oxydes et de leurs sels d'addition d'acides, caractérisé en ce que l'on fait réagir une 4-hydroxypipéridine de formule générale II ci-après:

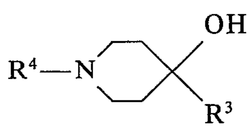

(II)

dans laquelle:

$R^3$ a la même signification que ci-dessus,

$R^4$ représente un groupe alkyle comportant de 1 à 5 atomes de carbone, un groupe alcényle comportant de 3 à 5 atomes de carbone, un groupe cycloalkylméthyle comportant de 3 à 7 atomes de carbone dans la partie cycloalkyle, un groupe phénylalkyle comportant de 1 à 3 atomes de carbone dans la partie alkyle ou un groupe $R^5-CO$, et

$R^5$ représente un atome d'hydrogène, un groupe alkyle comportant de 1 à 4 atomes de carbone, un groupe alcényle comportant de 2 à 4 atomes de carbone, un groupe cycloalkyle comportant de 3 à 7 atomes de carbone, un groupe phényle ou un groupe phénylalkyle comportant 1 ou 2 atomes de carbone dans la partie alkyle,

ou un sel d'une telle 4-hydroxypipéridine, avec du 4-nitrofluorobenzène, dans un solvant aprotique, en présence d'une base forte, et en ce que, le cas échéant, on soumet unsuite le produit de cette réaction à l'une ou plusieurs des étapes de procédé suivantes:

a) désalkylation de composés de formule générale I obtenus, dans lesquels $R^1$ est un groupe alkyle, alcényle, cycloalkylméthyle ou phénylalkyle conforme à la définition, pour obtenir des composés de formule générale I, dans lesquels $R^1$ est un atome d'hydrogène;
b) désacylation de composés de formule générale I obtenus, dans lesquels $R^1$ est un groupe $R^5-CO$, pour obtenir des composés de formule générale I, dans lesquels $R^1$ est un atome d'hydrogène;
c) alkylation de composés de formule générale I obtenus, dans lesquels $R^1$ est un atome d'hydrogène, pour obtenir des composés de formule générale I dans lesquels $R^1$ est un groupe alkyle, alcényle, cycloalkylméthyle ou phénalkyle conforme à la définition;
d) réduction de composés de formule générale I obtenus, dans lesquels $R^1$ est un groupe $R^5-CO$, pour obtenir des composés de formule générale I dans lesquels $R^1$ est un groupe alkyle, alcényle, cycloalkylméthyle ou phénalkyle conforme à la définition;
e) réduction de composés de formule générale I obtenus, dans lesquels $R^2$ est un groupe nitro-, pour obtenir des composés de formule générale I, dans lesquels $R^2$ est un groupe amino-, puis, le cas échéant, acylation, »acyl« ayant la signification donnée dans la définition;
f) réduction de composés de formule générale I obtenus, dans lesquels $R^2$ est un groupe amino-, pour obtenir des composés de formule générale I, dans lesquels $R^2$ est un atome d'hydrogène;
g) transformation de la base obtenue en un sel d'addition d'acide ou transformation d'un sel d'addition d'acide obtenu en base;
h) transformation de la base obtenue ou d'un sel d'addition d'acide obtenu en N-oxyde.

**Revendications pour l'Etat contractant: AT**

1. Procédé pour la préparation des 4-phénoxypipéridines de formule générale I ci-après:

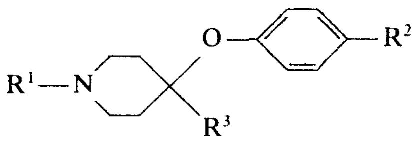

(I)

dans laquelle:

$R^1$ représente un atome d'hydrogène, un groupe alkyle comportant de 1 à 5 atomes de carbone, un groupe alcényle comportant de 3 à 5 atomes de carbone, un groupe cycloalkylméthyle comportant de 3 à 7 atomes de carbone dans la partie cycloalkyle ou un groupe phénylalkyle comportant de 1 à 3 atomes de carbone dans la partie alkyle,

$R^2$ représente un atome d'hydrogène, un groupe nitro-, un groupe amino- ou un groupe acylamino-, »acyl« ayant la signification d'un groupe alkylcarbonyle comportant 1 à 5 atomes de carbone dans la partie alkyle, d'un groupe alcoxycarbonyle comportant 1 ou 2 atomes de carbone dans la partie alcoxy, ou d'un groupe benzoyl éventuellement substitué par des atomes de chlore, de brome, de fluor, des groupes méthyle, trifluorméthyle ou méthoxy-, et

$R^3$ représente un groupe phényle ou benzyle,

ainsi que de leurs N-oxydes et de leurs sels d'addition d'acides, caractérisé en ce que l'on fait réagir une 4-hydroxypipéridine de formule générale II ci-après:

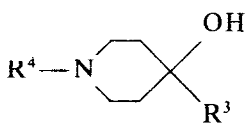

(II)

dans laquelle:

$R^3$ a la même signification que ci-dessus,

$R^4$ représente un groupe alkyle comportant de 1 à 5 atomes de carbone, un groupe alcényle comportant de 3 à 5 atomes de carbone, un groupe cycloalkylméthyle comportant de 3 à 7 atomes de carbone dans la partie cycloalkyle, un groupe phénylalkyle comportant de 1 à 3 atomes de carbone dans la partie alkyle ou un groupe $R^5-CO$, et

$R^5$ représente un atome d'hydrogène, un groupe alkyle comportant de 1 à 4 atomes de carbone, un groupe alcényle comportant de 2 à 4 atomes de carbone, un groupe cycloalkyle comportant de 3 à 7 atomes de carbone, un groupe phényle ou un groupe phénylalkyle comportant 1 ou 2 atomes de carbone dans la partie alkyle,

ou un sel d'une telle 4-hydroxypipéridine, avec du 4-nitrofluorobenzène, dans un solvant aprotique, en présence d'une base forte, et en ce que, le cas échéant, on soumet ensuite le produit de cette réaction à l'une ou plusieurs des étapes de procédé suivantes:

a) désalkylation de composés de formule générale I obtenus, dans lesquels $R^1$ est un groupe alkyle, alcényle, cycloalkylméthyle ou phénylalkyle conforme à la définition, pour obtenir des composés de formule générale I, dans lesquels $R^1$ est un atome d'hydrogène;
b) désacylation de composés de formule générale I obtenus, dans lesquels $R^1$ est un groupe $R^5-CO$, pour obtenir des composés de formule générale I, dans lesquels $R^1$ est un atome d'hydrogène;
c) alkylation de composés de formule générale I obtenus, dans lesquels $R^1$ est un atome d'hydrogène, pour obtenir des composés de formule générale I dans lesquels $R^1$ est un groupe alkyle, alcényle, cycloalkylméthyle ou phénalkyle conforme à la définition;
d) réduction de composés de formule générale I obtenus, dans lesquels $R^1$ est un groupe $R^5-CO$, pour obtenir des composés de formule générale I dans lesquels $R^1$ est un groupe alkyle, alcényle, cycloalkylméthyle ou phénalkyle conforme à la définition;
e) réduction de composés de formule générale I obtenus, dans lesquels $R^2$ est un groupe nitro-, pour obtenir des composés de formule générale I, dans lesquels $R^2$ est un groupe amino-, puis, le cas échéant, acylation, »acyl« ayant la signification donnée dans la définition;
f) réduction de composés de formule générale I obtenus, dans lesquels $R^2$ est un groupe amino-, pour obtenir des composés de formule générale I, dans lesquels $R^2$ est un atome d'hydrogène;
g) transformation de la base obtenue en un sel d'addition d'acide ou transformation d'un sel d'addition d'acide obtenu en base;
h) transformation de la base obtenue ou d'un sel d'addition d'acide obtenu en N-oxyde.

2. Procédé selon la revendication 1, caracterisé en ce qu'on prepare des 4-phénoxypipéridines de la formule générale I* ci-après:

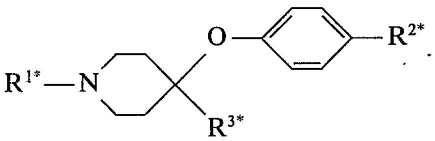

(I*)

dans laquelle:

$R^{1*}$ représente un atome d'hydrogène, un groupe alkyle à chaîne droite comportant de 1 à 3 atomes de carbone, ou un groupe phénylalkyle comportant 1 ou 2 atomes de carbone dans la partie alkyle,
$R^{2*}$ représente un atome d'hydrogène, un groupe nitro-, un groupe amino- ou un groupe alkylcarbonylamino- comportant de 1 à 5 atomes de carbone, et,
$R^{3*}$ représente un groupe phényle ou benzyle,

ainsi que leurs N-oxydes et leurs sels d'addition d'acides par reaction d'une 4-hydroxypipéridine de la formule générale II* ci-après:

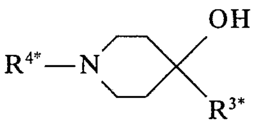

(II*)

dans laquelle:

$R^{3*}$ a la même signification que ci-dessus,
$R^{4*}$ représente un groupe alkyle comportant de 1 à 3 atomes de carbone, un groupe phénylalkyle comportant de 1 ou 2 atomes de carbone dans la partie alkyle ou un groupe $R^{5*}-CO$, et
$R^{5*}$ représente un atome d'hydrogène, un groupe alkyle comportant de 1 ou 2 atomes de carbone, un groupe phényle ou un groupe benzyle,

ou un sel d'une telle 4-hydroxypipéridine, avec du 4-nitrofluorobenzène, dans un solvant aprotique, en présence d'une base forte, et en ce que, le cas échéant, on soumet ensuite le produit de cette réaction à l'une ou plusieurs des étapes de procédé suivantes:

a) désalkylation de composés de formule générale I obtenus, dans lesquels $R^{1*}$ est un groupe alkyle ou phénalkyle conforme à la définition, pour obtenir des composés de formule générale I*, dans lesquels $R^{1*}$ est un atome d'hydrogène;
b) désacylation de composés de formule générale I* obtenus, dans lesquels $R^{1*}$ est un groupe $R^{5*}-CO$, pour obtenir des composés de formule générale I*, dans lesquels $R^{1*}$ est un atome d'hydrogène;
c) alkylation de composés de formule générale I* obtenus, dans lesquels $R^{1*}$ est un atome d'hydrogène, pour obtenir des composés de formule générale I* dans lesquels $R^{1*}$ est un groupe alkyle ou phénalkyle conforme à la définition;
d) réduction de composés de formule générale I* obtenus, dans lesquels $R^{1*}$ est un groupe $R^{5*}-CO$, pour obtenir des composés de formule générale I* dans lesquels $R^{1*}$ est un groupe alkyle ou phénalkyle conforme à la définition;
e) réduction de composés de formule générale I* obtenus, dans lesquels $R^{2*}$ est un groupe nitro-, pour obtenir des composés de formule générale I*, dans lesquels $R^{2*}$ est un groupe amino-, puis, le cas échéant, acylation, »acyl« ayant la signification d'alkylcarbonyl donnée ci-dessus;
f) réduction de composés de formule générale I* obtenus, dans lesquels $R^{2*}$ est un groupe amino-, pour obtenir des composés de formule générale I*, dans lesquels $R^{2*}$ est un atome d'hydrogène;
g) transformation de la base obtenue en un sel d'addition d'acide ou transformation d'un sel d'addition d'acide obtenu en base;
h) transformation de la base obtenue ou d'un sel d'addition d'acide obtenue en N-oxyde.

3. Procédé selon la revendication 2, caracterisé en ce qu'on prepare des 4-phénoxypipéridines de la formule I*, dans lesquelles $R^{1*}$ est un atome d'hydrogène, un groupe méthyle ou benzyle, $R^{2*}$ est un atome d'hydrogène, un groupe nitro- ou un groupe amino- et $R^{3*}$ est un groupe phényle ou benzyle, leurs N-oxydes ou leurs sels d'addition d'acides.

4. Procédé selon la revendication 2, caracterisé en ce qu'on prepare des 4-phénoxypipéridines de la formule I*, dans lesquelles $R^{1*}$ est un atome d'hydrogène, un groupe méthyle ou benzyle, $R^{2*}$ est un

atome d'hydrogène, un groupe nitro- ou un groupe amino- et $R^{3*}$ est un groupe phényle, ou leurs sels d'addition d'acides pharmacologiquement compatibles.

5. Procédé selon la revendication 2, caracterisé en ce qu'on prepare des 4-phénoxypipéridines de la formule I*, dans lesquelles $R^{1*}$ est un atome d'hydrogène, un groupe méthyle ou un groupe benzyle, $R^{2*}$ est un groupe amino- et $R^{3*}$ est un groupe phényle, ou leurs sels d'addition d'acides pharmacologiquement compatibles.

6. Procédé selon la revendication 2, caracterisé en ce qu'on prepare la 4-phénoxypipéridine de la formula I*, dans laquelle $R^{1*}$ est un groupe méthyle, $R^{2*}$ est un groupe amino- et $R^{3*}$ est un groupe phényle, ou ses sels d'addition d'acides pharmacologiquement compatibles.

7. Procédé selon la revendication 1, caracterisé en ce qu'on prepare des 4-phénoxypipéridines de la formule générale I** ci-après:

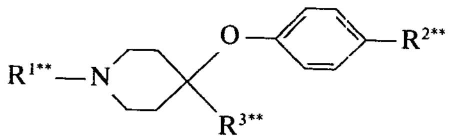

(I**)

dans laquelle:

$R^{1**}$ représente un groupe alkyle à chaîne ramifiée comportant de 3 à 5 atomes de carbone, un groupe alcényle comportant de 3 à 5 atomes de carbone ou un groupe cycloalkylméthyle comportant de 3 à 5 atomes de carbone dans la partie cycloalkyle,
$R^{2**}$ représente un atome d'hydrogène, un groupe nitro-, un groupe amino- ou un groupe alkylcarbonylamino- comportant de 1 à 5 atomes de carbone, et
$R^{3**}$ représente un groupe phényle ou benzyle,

ainsi que leurs N-oxydes et leurs sels d'addition d'acides par reaction d'une 4-hydroxypipéridine de formule générale II** ci-après:

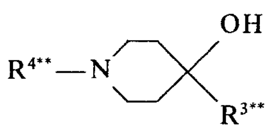

(II**)

dans laquelle:

$R^{3**}$ a la même signification que ci-dessus,
$R^{4**}$ représente un groupe alkyle à chaîne ramifieé comportant de 3 à 5 atomes de carbone, un groupe alcényle comportant de 3 à 5 atomes de carbone, un groupe cycloalkylméthyle comportant de 3 à 5 atomes de carbone dans la partie cycloalkyle ou un groupe $R^{5**}-CO$, et
$R^{5**}$ représente un groupe alkyle comportant de 2 à 4 atomes de carbone, un groupe alcényle comportant de 2 à 4 atomes de carbone ou un groupe cycloalkyle comportant de 3 à 5 atomes de carbone,

ou un sel d'une telle 4-hydroxypipéridine, avec du 4-nitrofluorobenzène, dans un solvant aprotique, en présence d'une base forte, et en ce que, le cas échéant, on soumet ensuite le produit de cette réaction à l'une ou plusieurs des étapes de procédé suivantes:

a) désalkylation de composés de formule générale I** obtenus, dans lesquels $R^{1**}$ est un groupe alkyle, alcényle ou cycloalkylméthyle conforme à la définition, pour obtenir des composés de formule générale I**, dans lesquels $R^{1**}$ est un atome d'hydrogène;
b) désacylation de composés de formule générale I** obtenus, dans lesquels $R^{1**}$ est un groupe $R^{5**}-CO$, pour obtenir des composés de formule générale I**, dans lesquels $R^{1**}$ est un atome d'hydrogène;
c) alkylation de composés de formule générale I** obtenus, dans lesquels $R^{1**}$ est un atome d'hydrogène, pour obtenir des composés de formule générale I** dans lesquels $R^{1**}$ est un groupe alkyle, alcényle ou cycloalkylméthyle conforme à la définition;
d) réduction de composés de formule générale I** obtenus, dans lesquels $R^{1**}$ est un groupe $R^{5**}-CO$, pour obtenir des composés de formule générale I** dans lesquels $R^{1**}$ est un groupe alkyle, alcényle ou cycloalkylméthyle conforme à la définition;
e) réduction de composés de formule générale I** obtenus, dans lesquels $R^{2**}$ est un groupe nitro-, pour obtenir des composés de formule générale I**, dans lesquels $R^{2**}$ est un groupe amino-, puis, les cas échéant, acylation, »acyl« ayant la signification d'alkylcarbonyl donnée ci-dessus;

f) réduction de composés de formule générale I** obtenus, dans lesquels $R^{2**}$ est un groupe amino-, pour obtenir des composés de formule générale I**, dans lesquels $R^{2**}$ est un atome d'hydrogène;
g) transformation de la base obtenue en un sel d'addition d'acide ou transformation d'un sel d'addition d'acide obtenu en base;
h) transformation de la base obtenue ou d'un sel d'addition d'acide obtenu en N-oxyde.

8. Procédé selon la revendication 7, caracterisé en ce qu'on prepare des 4-phénoxypipéridines de la formule I**, dans lesquelles $R^{1**}$ est un groupe alkyle à chaîne ramifiée comportant de 3 à 5 atomes de carbone, un groupe allyle ou cyclopropylméthyle, $R^{2**}$ est un atome d'hydrogène, un groupe nitro- ou un groupe amino- et $R^{3**}$ est un groupe phényle ou benzyle, leurs N-oxydes ou leurs sels d'addition d'acides.

9. Procédé selon la revendication 7, caracterisé en ce qu'on prepare des 4-phénoxypipéridines de la formule I** dans lesquelles $R^{1**}$ est un groupe isopropyle ou butyle tertiaire, $R^{2**}$ est un atome d'hydrogène, un groupe nitro- ou un groupe amino- et $R^{3**}$ est un groupe phényle, ou leurs sels d'addition d'acides pharmacologiquement compatibles.